# EUROPEAN PATENT SPECIFICATION

(11) **EP 2 961 369 B1**
(45) Date of publication and mention of the grant of the patent: **12.09.2018**
(21) Application number: 14723608.7
(22) Date of filing: 01.04.2014
(51) Int. Cl.: A61K 36/48, A61K 8/9789, A61Q 5/12, A61Q 19/00, A61Q 17/04

(54) **COMPOSITIONS OF DELIVERY SYSTEMS AND LUPINE WITH PHYSICAL STABILITY**
ZUSAMMENSETZUNGEN AUS ABGABESYSTEMEN UND LUPINEN MIT PHYSIKALISCHER STABILITÄT
COMPOSITIONS CONTENANT DES SYSTÈMES DE LIBÉRATION ET DU LUPIN PRÉSENTANT UNE BONNE STABILITÉ PHYSIQUE

(30) Priority: 02.04.2013 US 201361807564 P
(43) Date of publication of application: 06.01.2016
(73) Proprietor: Evonik Schlüchtern GmbH, 36381 Schlüchtern (DE)
(72) Inventor: BEYER, Monika, 61130 Nidderau-Ostheim (DE); GETZAT, Caroline, 36381 Schlüchtern (DE); SACHER, Michael Manfred, 36381 Schluchtern-Breitenbach (DE)
(74) Representative: Herrmann, Lutz
(86) International application number: PCT/US2014/032485
(87) International publication number: WO 2014/165484

(56) References cited:
- WO-A2-03/070172
- FR-A1- 2 616 659
- US-A- 4 892 727
- US-A1- 2005 148 498
- US-A1- 2005 266 065
- US-A1- 2012 156 272
- US-A1- 2012 195 948
- US-A1- 2012 251 614
- DATABASE GNPD [Online] MINTEL; 1 February 2013 (2013-02-01), Amway: "Eye cream", XP002732212, Database accession no. 1991917
- Laboratoires Expanscience: "alpha-Lupaline (TM)", France , 5 September 2011 (2011-09-05), pages 1-1, XP002732213, www.archive.org Retrieved from the Internet: URL:https://web.archive.org/web/2011090501 1851/http://www.expanscience-ingredients.c om/pdf/en/lupaline.pdf [retrieved on 2014-11-06]
- Sofia Sipsas ET AL: "LUPIN PRODUCTS - CONCEPTS AND REALITY", International Lupin Association, 1 January 2008 (2008-01-01), pages 506-513, XP055151457, Retrieved from the Internet: URL:http://www.lupins.org/pdf/conference/2 008/Food and Health/Sofia Sipsa.pdf [retrieved on 2014-11-06]

## Description

This Application claims the benefit of U.S. Application No. 61/807,564, filed on 02 April 2013. The disclosure of Application No. 61/807,564 is hereby incorporated by reference.

### BACKGROUND OF THE INVENTION

The present disclosure relates generally to the field of cosmetic actives and delivery systems and more particularly to compositions comprising a lupine composition and a delivery system and their use in personal care compositions.

It is known that a variety of botanical substances are used in the preparation of cosmetic and other personal care products. These botanical substances can provide a range of functionality (e.g.: cosmetic actives, emollients, emulsifiers, among others) and benefits as cosmetic ingredients. Various substances obtained from the lupine plant are included among these botanical substances.

To obtain the desired functionality and benefits from the various substances derived from the lupine plant, a number of processes may be employed, such as extraction, purification, concentration, and enzymatic hydrolysis, as in US7,638,149 and US2008050458. While such processes may be useful to increase the purity or modify the functionality of the lupine substance, they can also add cost to the cosmetic ingredient.

Use of a simple ground powder of lupine seeds in cosmetic compositions is disclosed in US4,892,727. WO9911143 and WO9951106 disclose certain lupine proteins that are used to stabilize certain oil and water emulsions.

The disclosure of the previously identified patents and patent publications is hereby incorporated by reference.

### BRIEF SUMMARY OF THE INVENTION

The instant invention can overcome these and other drawbacks by providing compositions of Lupine Compositions (defined below) and a Delivery System (hereinafter Lupine-Based Delivery System) that are physically stable, wherein at least a portion of a Lupine Composition is encapsulated in the delivery system. These compositions provide desirable characteristics for use in Personal Care compositions such as moisturization of keratinous substrates, including Cosmetics (defined below).

The present invention also relates to a method for preparing compositions that are physically stable comprising a Lupine Composition and a Delivery System, wherein at least a portion of the Lupine Composition is encapsulated in the Delivery System. Further, the present invention relates to methods for using the Delivery System in order to obtain compositions that are physically stable (defined below) comprising a Lupine Composition and Delivery System, obtainable by or obtained by said method. Furthermore, the present invention relates to compositions that are physically stable comprising a Lupine Composition and Delivery System, wherein at least a portion of, for example, a Lupine Powder is encapsulated in the Delivery System, for use in Personal Care products including Cosmetics. When used in Personal Care compositions for skin care, the Lupine-Based Delivery System can improve skin moisturization, and may offer additional benefits to dry skin of improved skin smoothness, improved healthy appearance, reduced redness, reduced itching, and reduced scaliness. The following definitions are provided and used throughout this disclosure:
"Lupine" means and comprises any plant within the genus Lupinus including, without limitation, Lupinus albus, and includes the entire plant, root, stem, seeds, flowers, leaves and any other constituents of the plant. Lupine also means constituent proteins, fibers, polysaccharides, and lipids of the Lupine plant.
"Lupine Composition" means and comprises Lupine Powder, Lupine Extract, and materials containing or derived from any one or more parts of, or whole Lupine plant including, without limitation material derived from chemical or physical processes including, without limitation, hydrolyzed extracts, partially hydrolyzed extracts, or combinates thereof, extraction, grinding, pulverizing, milling, among other processes.
"Lupine Powder" means and comprises particulate material obtained from Lupine, and includes a particulate material obtained from Lupine seeds and, can include, material from other parts of the Lupine plant.
"Lupine Extract" means and comprises the contact product of at least one solvent and a Lupine, and includes an extract obtained from Lupine seeds.
"Delivery System" means and comprises at least one of Cerasome, Liposome I, and Liposome II including, without limitation, phospholipid, ceramide, glycosphingolipid, solvent and other ingredients of the System.
"Lupine-Based Delivery System" means and comprises a Lupine Composition combined with a Delivery System and includes, without limitation, compositions of the Lupine Composition and the Delivery System that is physically stable (defined below).
"Personal Care" means and comprises any toiletry and topical care products including, without limitation, leave-on personal care products (i.e., products that are left on keratinous substrates after application); rinse-off personal care products (i.e., products that are washed or rinsed from keratinous substrates during or within a few minutes of application); at least one of shampoos; chemical and non-chemical hair curling and hair straightening products; hair style maintaining and hair conditioning products; lotions and creams for nails, hands, feet, face, scalp and/or body; hair dye; face and body makeup; nail care products; astringents; deodorants; antiperspirants; antiacne; antiaging; depilatories; colognes and perfumes; skin protective creams and lotions (such as sunscreens); skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products). The form of such Personal Care products is not limited and may be, without limitation, a liquid, gel, spray, emulsion (such as lotions and creams), shampoo, pomade, foam, tablet, stick (such as lip care products), makeup, suppositories, among others, any of which can be applied to the skin or hair and which typically are designed to remain in contact there with until removed, such as by rinsing with water or washing with shampoo or soap. Other forms could be gels that can be soft, stiff, or squeezable. Emulsions can be oil-in-water, water-in-oil, water-and-silicone, or multiphase. Sprays can be non-pressurized aerosols delivered from manually pumped finger-actuated sprayers or can be pressurized aerosols such as mousse, spray, or foam forming formulation, where a chemical or gaseous propellant is used.
"Cosmetic" means and comprises a Personal Care product comprising a powder, lotion, lipstick, rouge, or other preparation for affecting the appearance of or beautifying the face, skin, hair, nails or other keratinous materials.
"Physical stability" or "Physically stable" means that a given composition has not formed two visually distinct and separated layers after being heated at an elevated temperature (e.g., 30 days at 40 °C or 15 days at 50°C).

One embodiment of the invention relates to a composition comprising at least one Lupine Composition and at least one Delivery System according to claim 1.

Another embodiment of the invention relates to a Lupine Composition that comprises a Lupine Extract.

Another embodiment of the invention relates to a Lupine composition wherein the Lupine Extract comprises a seed extract of Lupinus albus.

In one embodiment of the invention, the Lupine Composition comprises Lupine Powder.

In another embodiment of the invention, the Lupine Powder comprises a seed of genus Lupinus.

In a further embodiment of the invention, the seed comprises Lupinus albus.

In one embodiment of the invention, the Lupine Composition comprises materials derived from any one or more parts of, or whole plant of, any plant within genus Lupinus.

In another embodiment of the invention, the foregoing material comprises at least one of hydrolyzed Lupine extracts, partially hydrolyzed Lupine extracts, a substance derived from lupine and combinations thereof.

According to the present invention, the Delivery System comprises at least one member selected from the group consisting of Cerasome, Liposome I and Liposome II.

In one embodiment of the invention, the Delivery System comprises Liposome I, and the Liposome I comprises at least one of Phospholipids of type PL2.

In one embodiment of the invention, the composition further comprises at least one solvent selected from the group consisting of ethanol, 1,2-propanediol, 1,3-propanediol, 1,3-butylene glycol, and glycerol.

In another embodiment of the invention, the Lupine Based Delivery System is employed for making a Personal Care product as well as Cosmetic products.

In any one of the foregoing embodiments, the Lupine Based Delivery System is physically stable.

In another embodiment of the invention, the composition further comprises at least one chelating agent.

In a further embodiment of the invention, the composition further comprises at least one preservative.

Another embodiment of the invention relates to using at least one Delivery System to yield a Lupine-Based Delivery System composition that is physically stable.

The various embodiments of the invention can be used alone or in combination.

### DETAILED DESCRIPTION OF THE INVENTION

The present invention is directed to compositions that are physically stable comprising a Lupine Composition and a Delivery System according to claim 1, wherein at least a portion of, for example, a Lupine Powder is encapsulated in the Delivery System. The Delivery System of the present invention comprises vesicular compositions including membrane-forming lipids such as double-layer membrane vesicles. Liposomes are an example of the double-layer and also multi-layer vesicles. The Lupine Composition can be present both in the interior of the vesicles in a solution or dispersion, and also incorporated into or between the layers. The membrane-forming lipids of the Delivery System comprise the membrane-forming lipids from the groups of naturally-derived or synthetic phospholipids, naturally-derived or synthetic ceramides and naturally-derived or synthetic glycosphingolipids, and mixtures thereof. The Lupine Composition is combined with the Delivery System in order to obtain a Lupine-Based Delivery System which in turn can be combined with other ingredients to produce a Personal Care product.

### Lupine Compositions

According to one embodiment of this invention, the Lupine Composition comprises a "Lupine Powder" of the genus Lupinus that comprised proteins, polysaccharides, lipids, fibers and, optionally water, wherein the amount of protein was about 30 wt% to about 70 wt%, and in some cases from about 35 wt% to about 60 wt%, and in other cases from about 40 wt% to about 50 wt%, based on the total weight of the "Lupine Powder", and wherein the amount of polysaccharides was about 1 wt% to about 25 wt%, and in some cases from about 5 wt% to about 20 wt%, and in other cases from about 7 wt% to about 15 wt%, based on the total weight of the "Lupine Powder", and wherein the amount of lipids was about 1 wt% to about 25 wt%, and in some cases from about 5 wt% to about 20 wt%, and in other cases from about 7 wt% to about 15 wt%, based on the total weight of the "Lupine Powder", and wherein the amount of fibers was about 2 wt% to about 35 wt%, in some cases about 5 wt% to about 35 wt%, and in other cases about 25 wt% to about 35 wt%, based on the total weight of the "Lupine Powder", and wherein the amount of water was preferably less than about 15 wt%, based on the total weight of the "Lupine Powder".

In another embodiment, the "Lupine Powder" comprises proteins, polysaccharides, lipids, fibers, and optionally water in the aforementioned amounts, and was from the species Lupinus albus.

In other embodiments, the "Lupine Powder" comprises proteins, polysaccharides, lipids, fibers, and optionally water in the aforementioned amounts, and is obtained from any Lupinus species including, but not limited to, Lupinus lutens, Lupinus subcarnosus and Lupinus texensis. In still other embodiments, the "Lupine Powder" is a powder derived from any Lupinus species including, but not limited to, Lupinus albus, Lupinus lutens, Lupinus subcarnosus and Lupinus texensis. Thus, as used herein, the term "Lupine" means any type of lupine including, but not limited to, any cultivated lupine, any wild lupine, any lupine species, any intra-and interspecies lupine crosses, all lupine varieties, all lupine genotypes and all lupine cultivars.

In certain embodiments, the Lupine Powder comprises fine, loose, solid particles with a mean particle diameter of from about 0.25 to about 500 microns, in some cases from about 2 to about 350 microns, and in other cases from about 10 to about 200 microns as determined by any suitable method, such as a laser diffraction particle size analyzer, including but not limited to a Microtrac S3500 using methods described in its operating manual.

In another embodiment of the invention the Lupine Composition comprises "Lupine Extract" obtained from a Lupine which is obtained by extraction, maceration or percolation of the Lupine with a suitable solvent and, optionally, by partial or complete removal of the solvent. Thus, extracts in accordance with this invention are either so-called solvent-processed fluid extracts or so-called dry Lupine extracts obtained by evaporation of the whole liquid extract to dryness, e.g. by air drying, spray drying, vacuum oven drying, fluid-bed drying or freeze-drying, and optional washing and/or re-dissolving of this dry extract in at least one suitable solvent using equipment and procedures known to those in this art. In embodiments where a dry Lupine extract is used, the Lupine Powder comprises fine, loose, solid particles with a mean particle diameter of from about 0.25 to about 500 microns, in some cases from about 2 to about 350 microns, and in other cases from about 10 to about 200 microns as determined by any suitable method, such as a laser diffraction particle size analyzer, including but not limited to a Microtrac S3500 using methods described in its operating manual.

Solvents suitable for extraction, percolation or maceration are known to those experienced in the art. Acetone, chloroform, ethyl acetate, lower alkanols with 1 to 4 carbon atoms, alcohols or a mixture of these and water are particularly suited. Carbon dioxide in fluid or super-critical form and pressurized gases with solvent properties are also suitable as extraction agents. In some embodiments the Lupine Powder extracts are obtained from the seeds of the lupine, and in other cases can be obtained from other parts of the plant including one or more of the leaves, stems, and roots, or of the entire plant.

In other embodiments, the "Lupine Extract" can comprise hydrolyzed extracts, partially hydrolyzed extracts and combinations thereof, that can be obtained as a liquid or solution by solvent-processed fluid extraction methods or as a dry lupine extracts obtained by evaporation of the whole liquid extract to dryness, e.g. by air drying, spray drying, vacuum oven drying, fluid-bed drying or freeze-drying, and optional washing and/or re-dissolving of this dry extract in at least one suitable solvent to yield a liquid extract.

Lupine Compositions are naturally occurring materials and as such include inherent variations that can affect their ability to be used for making Lupine-Based Delivery Systems. In particular, Lupine Compositions having relatively high concentrations of components that are insoluble in the Delivery System may fail to produce a physically stable Lupine-Based Delivery System (as determined by visual inspection). Examples of Lupine Compositions that fail to produce a physically stable composition when combined with a Delivery System, including but not limited to "Liposome I" and "Liposome II", consist of, but may not be limited to, Lupine Powders comprising protein in an amount from about 51 wt% to about 75 wt% and further comprising fibers in an amount from about 15 wt% to about 25 wt%. Accordingly, in order to obtain a physically stable Lupine-Based Delivery System it is important that compatible Lupine Compositions and Lupine Compositions be employed.

### Cerasome

In one embodiment of the invention, the Lupine Composition is combined with a Delivery System according to claim 1 comprising a Cerasome. The term "Cerasome" is denoted to mean an artificially prepared vesicle made of at least one lipid bilayer (lipid membrane), wherein the delivery system comprises naturally-derived or synthetic ceramides and naturally-derived or synthetic glycosphingolipids, which structure can act as a physical reservoir for active ingredients such as for the Lupine Composition.

In a first embodiment, the composition comprises a Lupine Composition and a "Cerasome" delivery system. Compositions of the first embodiment that are physically stable (visually determined) and with desirable characteristics for use in Cosmetic or Personal Care compositions are comprised of at least:
i) one or more naturally-derived or synthetic ceramides and/or naturally-derived or synthetic glycosphingolipids in an amount from about 0.1 wt% to about 15 wt%, in some cases from about 0.5 wt% to about 8 wt%, and in other cases from about 1 wt% to about 4 wt%, based on the total weight of the composition,
ii) at least one Lupine Composition in an amount from about 0.1 wt% to about 15 wt%, in some cases from about 0.5 wt% to about 10 wt%, and in other cases from about 1 wt% to about 4 wt%, based on the total weight of the composition,
iii) at least one solvent, comprising at least one alcohol, glycol, or triol, in an amount from about 5 wt% to about 30 wt%, in some cases from about 8 wt% to about 25 wt%, and in other cases from about 10 wt% to about 20 wt%, based on the total weight of the composition,
iv) water in an amount of at least 30 wt%, in some cases at least 50 wt%, and in other cases at least 60 wt%, based on the total weight of the composition,
v) and, optionally, one or more Other Functional Ingredients (defined below).

Specific examples of suitable solvents comprise at least one member selected from the group consisting of ethanol, 1,2-propanediol, 1,3-propanediol, 1,3-butylene glycol, and glycerol. In some cases the solvent comprises ethanol in an amount from about 5 to about 30 wt%, and in other cases from about 8 wt% to about 25 wt%.

In certain embodiments, the inventive composition is a Lupine-Based Delivery System that comprises a Lupine Composition and a "Cerasome" Delivery System according to claim 1. The inventive composition is obtained by combining the following ingredients: one or more naturally-derived or synthetic ceramides and/or naturally-derived or synthetic glycosphingolipids, at least one Lupine Composition, at least one solvent comprising at least one alcohol, glycol, or triol; and water. The combined ingredients comprise greater than about 75 wt%, in some cases greater than about 90 wt%, in other cases greater than about 95 wt%, and in still other cases greater than about 97.5 wt%, based on the total weight of the composition. If desired, the compositions of the instant invention can be used as an ingredient in a Cosmetic or Personal Care product (e.g., the inventive composition is combined with other materials in order to form a Cosmetic or Personal Care product).

In some embodiments of a Lupine Composition and a "Cerasome" delivery system, the Lupine Composition is a Lupine Powder.

Lupine-Based Delivery Systems that employ "Cerasome" compositions of the invention may be made by mixing together an aqueous phase containing, for example, the Lupine Powder and a ceramide or glycosphingolipids solution according to any suitable method described in the prior art, or known to those skilled in the art. In a first process step, the Lupine Powder is dispersed in water. In certain embodiments, during the first step the Lupine Powder is heated in water at a temperature of about 50 °C to about 90 °C, and in some cases from about 70 °C to about 80 °C, for a period of about 15 minutes to about 120 minutes, and in other cases from about 30 minutes to about 60 minutes, and then in some cases allowed to cool to room temperature. Without being bound to explanation, it is believed that in some cases this step increases the fraction of water soluble material in the Lupine Powder and may thus decrease the tendency for sediment to form in the inventive compositions. In a second process step the Ceramides or Glycosphingolipids are dissolved in alcohol, glycol, triol, or a combination thereof, at a temperature of about 30 °C to about 80 °C, and in some cases from about 40 °C to about 60 °C. Finally the aqueous phase containing the Lupine Powder and the Ceramide or Glycosphingolipids solution are mixed together at ambient temperature while stirring and homogenizing by means of a high shear homogenizer, non-limiting examples of which include high shear rotor-stator type homogenizers, high pressure homogenizers and ultrasonic homogenizers, for a period of about 2 minutes to about 90 minutes, and in some cases from about 5 minutes to about 60 minutes.

In other embodiments, Other Functional Ingredients can optionally be incorporated by adding and dissolving them in the aqueous phase, or by adding and dissolving them in the solution containing the Ceramides or Glycosphingolipids, depending on the solubility characteristics of the Other Functional Ingredients, at a temperature of about 20 °C to about 80 °C, and in some cases from about 40 °C to about 60 °C, and in other cases at ambient temperature prior to the mixing and homogenizing step.

The aforementioned at least one lipid bilayer of the "Cerasome" according to the invention may lead to a multilamellar (composed of more than one lipid bilayer) or an unilamellar structure (composed of only one lipid bilayer), wherein unilamellar vesicles are preferred. According to further embodiments of the invention, the average diameter (particle size, herein also referred to as "diameter") of the "Cerasome " is from about 50 nm to about 500 nm, in some cases from about 100 nm to about 400 nm, and in yet other cases from about 150 nm to about 300 nm, as measured by Photon Correlation Spectroscopy (PCS). "Particle Size" or "diameter" refers to the averaged hydrodynamic diameter as measured from diluted aqueous solutions by Photon Correlation Spectroscopy, also referred to as Dynamic Light Scattering. The particle size of "Cerasome" was measured by Photon Correlation Spectroscopy using a MALVERN Zetasizer Nano SZ90 by means of a 5 mW He-Ne laser (633 nm), with a measuring angle of 90° at a temperature of 25 °C +/- 0.5 °C, according to the procedure published in the MALVERN operating manual.

Additionally, the present invention relates to use of an aforementioned method for preparing a composition that is physically stable, as described above, and a composition that is physically stable obtainable or obtained by an aforementioned method, wherein the "Cerasome" have a particle size from about 50 nm to about 500 nm, in some cases from about 100 nm to about 400 nm, and in yet other cases from about 150 nm to about 300 nm, as measured by Photon Correlation Spectroscopy as described previously.

### Liposome I

In one embodiment of the invention the Lupine Composition is combined with a Delivery System according to claim 1 comprising a Liposome I. The term "Liposome I" is denoted to mean an artificially prepared vesicle made of at least one lipid bilayer (lipid membrane), wherein the delivery system comprises naturally-derived or synthetic phospholipids of type PL2, which structure can act as a physical reservoir for active ingredients such as for the Lupine Composition. Phospholipids of type PL2 comprise phosphatidylcholine, phosphatidylethanolamine and phosphatidic acid, wherein the content of phosphatidylcholine is from about 10 wt% to about 60 wt%, and in some cases from about 15 wt% to about 45 wt%, and in yet other cases from about 20 wt% to about 35 wt%, based on the total weight of the phospholipids.

The composition according to claim 1 and comprising a Lupine Composition and a "Liposome I" delivery system that are physically stable (as determined visually) and with desirable characteristics for use in Cosmetic or Personal Care compositions are comprised of at least
i) one or more naturally-derived or synthetic phospholipids of type PL2, in an amount from about 0.1 wt% to about 25 wt%, in some cases from about 1 wt% to about 15 wt%, and in other cases from about 5 wt% to about 10 wt%, based on the total weight of the composition,
ii) at least one Lupine Composition in an amount from about 0.1 wt% to about 15 wt%, in some cases from about 0.5 wt% to about 10 wt%, and in other cases from about 1 wt% to about 4 wt%, based on the total weight of the composition,
iii) at least one solvent, comprising at least one alcohol, glycol or triol, in an amount from about 5 wt% to about 50 wt%, in some cases from about 8 wt% to about 35 wt%, and in other cases from about 10 wt% to about 25 wt%, based on the total weight of the composition,
iv) water in an amount of at least 30 wt%, in some cases at least 50 wt%, and in other cases at least 60 wt%, based on the total weight of the composition,
v) and, optionally, one or more Other Functional Ingredients.

In certain embodiments of inventive compositions comprising a "Liposome I", the solvent is selected from one or more of ethanol, 1,2-propanediol, 1,3-propanediol, 1,3-butylene glycol, and glycerol. Wherein the composition comprises ethanol, the amount is from about 5 wt% to about 15 wt%, based on the total weight of the composition. Wherein the composition comprises 1,2-propanediol or 1,3-propanediol, the amount is from about 15 wt% to about 25 wt%, based on the total weight of the composition. Wherein the composition comprises 1,3-butylene glycol, the amount is from about 5 wt% to about 25 wt%, based on the total weight of the composition. Wherein the composition comprises glycerol, the amount is from about 15 wt% to about 35 wt%, based on the total weight of the composition. While any one or more of ethanol, 1,2-propanediol, 1,3-propanediol, 1,3-butylene glycol, or glycerol can provide compositions that are physically stable and suitable for use in personal care, consumer acceptance of ethanol in skin care applications, particularly in face care applications has decreased in recent years, and similarly an increasing number of manufacturers of cosmetic or personal care products prefer to not use 1,2-propanediol or 1,3-propanediol as an ingredient. In some embodiments, the selection of alcohol, glycol or triol is from a plant-based or renewable source.

In certain embodiments, the inventive composition is a Lupine-Based Delivery System that comprises a Lupine Composition and a "Liposome I" Delivery System according to claim 1. The inventive composition is obtained by combining the following ingredients: one or more naturally-derived or synthetic phospholipids of type PL2, at least one Lupine Composition, at least one solvent, comprising at least one alcohol, glycol, or triol; and water. The combined ingredients comprise greater than about 75 wt%, in some cases greater than about 90 wt%, in other cases greater than about 95 wt%, and in still other cases greater than about 97.5 wt%, based on the total weight of the composition. If desired, the compositions of the instant invention can be used as an ingredient in a Cosmetic or Personal Care product (e.g., the inventive composition is combined with other materials in order to form a Cosmetic or Personal Care product).

In some embodiments of a Lupine Composition and a "Liposome I" delivery system, the Lupine Composition is a Lupine Powder.

A Lupine-Based Delivery System comprising "Liposome I" compositions of the invention may be made by mixing together an aqueous phase containing, for example, the Lupine Powder and a solution containing Phospholipids of type PL2 according to any suitable method described in the prior art, or known to those skilled in the art. In a first process step, the Lupine Powder is dispersed in water. In certain embodiments, during the first step the Lupine Powder is heated in water at a temperature of about 50 °C to about 90 °C, and in some cases from about 70 °C to about 80 °C, for a period of about 15 minutes to about 120 minutes, and in other cases from about 30 minutes to about 60 minutes, and then in some cases allowed to cool to room temperature. Without being bound to explanation, it is believed that in some cases this step increases the fraction of water soluble material in the Lupine Powder and may thus decrease the tendency for sediment to form in the inventive compositions. In a second process step, a portion of the Phospholipids of type PL2 are optionally mixed into the aqueous phase and homogenized by means of a high shear homogenizer, the optional portion from about 0 to about 99%, and in some cases from about 0 to about 90%, and in other cases about 0 to about 50%, of the total amount of Phospholipids of type PL2 in the composition. In a third process step, the balance of the Phospholipids of type PL2 are dissolved in alcohol, glycol, triol, or a combination thereof, at a temperature of about 20 °C to about 80 °C, and in some cases from about 40 °C to about 60 °C and in other cases at ambient temperature. Finally the aqueous phase containing the Lupine Powder and the solution containing Phospholipids of type PL2 dissolved in alcohol, glycol, triol, or a combination thereof are mixed together at ambient temperature while stirring and homogenizing by means of a high shear homogenizer, non-limiting examples of which include high shear rotor-stator type homogenizers, high pressure homogenizers and ultrasonic homogenizers, for a period of about 2 minutes to about 90 minutes, and in some cases from about 5 minutes to about 60 minutes.

In some embodiments, the membrane-forming lipids of "Liposome I" may optionally comprise further membrane stabilizing components such as cholesterol and/or phytosterol.

In other embodiments, Other Functional Ingredients can optionally be incorporated by adding and dissolving them in the aqueous phase, or by adding and dissolving them in the solution containing Phospholipids of type PL2, depending on the solubility characteristics of the Other Functional Ingredients, at a temperature of about 20 °C to about 80 °C, and in some cases from about 40 °C to about 60 °C, and in other cases at ambient temperature prior to the mixing and homogenizing step.

The aforementioned at least one lipid bilayer of the "Liposome I" according to the invention may lead to a multilamellar or an unilamellar structure, wherein unilamellar vesicles are preferred. According to further embodiments of the invention, the particle size of the "Liposome I" is from about 50 nm to about 600 nm, in some cases from about 150 nm to about 500 nm, and in yet other cases from about 200 nm to about 450 nm, as measured by Photon Correlation Spectroscopy as described previously.

Additionally, the present invention relates to use of an aforementioned method for preparing a composition that is physically stable, as described above, and a composition that is physically stable obtainable or obtained by an aforementioned method, wherein the "Liposome I" have a particle size from about 50 nm to about 600 nm, in some cases from about 150 nm to about 500 nm, and in yet other cases from about 200 nm to about 450 nm, as measured by Photon Correlation Spectroscopy as described previously.

### Liposome II

In another embodiment of the invention, a Lupine Composition is combined with a Delivery System according to claim 1 comprising a Liposome II. The term "Liposome II" is denoted to mean an artificially prepared vesicle made of at least one lipid bilayer (lipid membrane), wherein the delivery system comprises naturally-derived or synthetic phospholipids of type PL1, which structure can act as a physical reservoir for active ingredients such as for the Lupine Powder. Phospholipids of type PL1 comprise phosphatidylcholine, phosphatidylethanolamine and phosphatidic acid, wherein the amount of phosphatidylcholine is from about 70 wt% to about 97 wt%, and in some cases from about 73 wt% to about 95 wt%, based on the total weight of the phospholipids, and wherein in some cases the amount of phosphatidylethanolamine is less than about 7 wt%, based on the total weight of the phospholipids, and wherein in some cases the amount of phosphatidic acid is less than about 8 wt%, based on the total weight of the phospholipids.

The composition according to claim 1 and comprising a Lupine Composition and a "Liposome II" delivery system that are physically stable (as determined visually) and with desirable characteristics for use in Cosmetic or Personal Care compositions are comprised of at least
i) one or more naturally-derived or synthetic phospholipids of type PL1, in an amount from about 1 wt% to about 25 wt%, in some cases from about 5 wt% to about 15 wt%, and in other cases from about 7 wt% to about 12 wt%, based on the total weight of the composition,
ii) at least one Lupine Composition in an amount from about 0.1 wt% to about 15 wt%, in some cases from about 0.5 wt% to about 10 wt%, and in other cases from about 1 wt% to about 4 wt%, based on the total weight of the composition,
iii) at least one solvent, comprising at least one alcohol, glycol or triol, in an amount from about 5 wt% to about 50 wt%, in some cases from about 10 wt% to about 40 wt%, and in other cases from about 15 wt% to about 35 wt%, based on the total weight of the composition,
iv) water in an amount of at least 30 wt%, in some cases at least 50 wt%, and in other cases at least 60 wt%, based on the total weight of the composition,
v) and, optionally, one or more Other Functional Ingredients.

Examples of suitable solvents comprise at least one member selected from the group consisting of ethanol, 1,2-propanediol, 1,3-propanediol, 1,3-butylene glycol, and glycerol. Wherein the solvent comprises at least one selected solvent from amongst ethanol, 1,2-propanediol, 1,3-propanediol, 1,3-butylene glycol, and glycerol, in some embodiments the amount of the selected solvent is from about 5 wt% to about 25 wt%, based on the total weight of the composition. In other embodiments the at least one solvent in an amount from about 10 wt% to about 40 wt% comprises ethanol in an amount from about 10 wt% to about 20 wt% and a second solvent selected from 1,2-propanediol, 1,3-propanediol, 1,3-butylene glycol, or glycerol, and in some cases the second solvent is 1,3-butylene glycol or glycerol.

In certain embodiments, the inventive composition is a Lupine-Based Delivery System that comprises a Lupine Composition and a "Liposome II" Delivery System according to claim 1. The inventive composition is obtained by combining:one or more naturally-derived or synthetic phospholipids of type PL1, at least one Lupine Composition, at least one solvent, comprising at least one alcohol, glycol, or triol; and water. The combined ingredients comprise greater than about 75 wt%, in some cases greater than about 90 wt%, in other cases greater than about 95 wt%, and in still other cases greater than about 97.5 wt%, based on the total weight of the composition. If desired, the compositions of the instant invention can be used as an ingredient in a Cosmetic or Personal Care product (e.g., the inventive composition is combined with other materials in order to form a Cosmetic or Personal Care product).

In some embodiments of a Lupine Composition and a "Liposome II" delivery system, the Lupine Composition is a Lupine Powder.

A Lupine-Based Delivery System comprising "Liposome II" compositions of the invention may be made by mixing together an aqueous phase containing, for example, the Lupine Powder and a solution containing Phospholipids of type PL1 according to any suitable method described in the prior art, or known to those skilled in the art. In a first process step, the Lupine Powder is dispersed in water. In certain embodiments, during the first step the Lupine Powder is heated in water at a temperature of about 50 °C to about 90 °C, and in some cases from about 70 °C to about 80 °C, for a period of about 15 minutes to about 120 minutes, and in other cases from about 30 minutes to about 60 minutes, and then in some cases allowed to cool to room temperature. Without being bound to explanation, it is believed that in some cases this step increases the fraction of water soluble material in the Lupine Powder and may thus decrease the tendency for sediment to form in the inventive compositions. In a second process step the Phospholipids of type PL1 are dissolved in alcohol, glycol, triol, or a combination thereof, at a temperature of about 20 °C to about 80 °C, and in some cases from about 40 °C to about 60 °C and in other cases at ambient temperature. Finally the aqueous phase containing the Lupine Powder and the solution containing Phospholipids of type PL1 are mixed together at ambient temperature while stirring and homogenizing by means of a high shear homogenizer, non-limiting examples of which include high shear rotor-stator type homogenizers, high pressure homogenizers and ultrasonic homogenizers, for a period of about 2 minutes to about 90 minutes, and in some cases from about 5 minutes to about 60 minutes.

In some embodiments, the membrane-forming lipids of "Liposome II" may optionally comprise further membrane stabilizing components such as cholesterol and/or phytosterol.

In other embodiments, Other Functional Ingredients can optionally be incorporated by adding and dissolving them in the aqueous phase, or by adding and dissolving them in the solution containing Phospholipids of type PL1, depending on the solubility characteristics of the Other Functional Ingredients, at a temperature of about 20 °C to about 80 °C, and in some cases from about 40 °C to about 60 °C, and in other cases at ambient temperature prior to the mixing and homogenizing step.

The aforementioned at least one lipid bilayer of the "Liposome II" according to the invention may lead to a multilamellar or an unilamellar structure, wherein unilamellar vesicles are preferred. According to further embodiments of the invention, the particle size of the "Liposome II" is from about 50 nm to about 600 nm, in some cases from about 100 nm to about 450 nm, and in yet other cases from about 150 nm to about 350 nm, as measured by Photon Correlation Spectroscopy as described previously.

Additionally, the present invention relates to use of an aforementioned method for preparing a composition that is physically stable, as described above, and a composition that is physically stable obtainable or obtained by an aforementioned method, wherein the "Liposome II" have a particle size from about 50 nm to about 600 nm, in some cases from about 100 nm to about 450 nm, and in yet other cases from about 150 nm to about 350 nm, as measured by Photon Correlation Spectroscopy as described previously.

### Ceramides and Glycosphingolipids

Within the context of the present invention the term "ceramide" and "glycosphingolipid" refers to complex lipids with sphingosine or a similar base as the basic structure, so-called "Sphingolipids". They are important components of plant and animal cell membranes and contain three characteristic components: one molecule of fatty acid, one molecule of sphingosine or sphingosine derivative and a polar (head) group, which can sometimes be very large and complex. Sphingosine is one of about 30 long chain aminoalcohols which are found in different types of sphingolipids. In mammals, sphingosine (4-sphingenine) and dihydrosphingosine (sphinganine) are the most usual bases of sphingolipids; in higher plants and yeasts, it is phytosphingosine (4-hydroxysphinganine) and in marine invertebrates, they are doubly unsaturated bases such as 4,8-sphingadins. The sphingosine base is bonded to a long chain unsaturated or monounsaturated fatty acid containing 18 to 26 carbon atoms via an amide bond in its amino group. This compound, which contains two non-polar chains, is ceramide, the characteristic basic structure of all sphingolipids. Unless expressly otherwise given, in the context of this application the term "ceramide" will have the same meaning as the term "sphingolipid".

The various derivatives of sphingolipids or ceramides can be divided further into groups depending on their behavior and structural chemical features. The most usual sphingolipids in the tissues of higher animals are sphingomyelins, which contain phosphorylethanolamine or phosphorylcholine as their polar groups. Another group of sphingolipids contains one or more neutral sugars as the polar group; they thus have no electric charge and hence are termed "neutral glycosphingolipids". Cerebrosides are the simplest members of this group; they have only one monosaccharide in [beta]-glycosidic combination with their hydroxyl group as the polar group. D-galactose is present in the cerebrosides of the brain and nervous system; they are thus termed galactocerebrosides. Cerebrosides are also present in small amounts in non neutral tissues; here, they contain mainly D-glucose and hence are termed glucocerebrosides. Sulphate esters of galactocerbrosides are termed sulphatides, which are also present in brain tissue. Cerebrosides and sulphatides contain fatty acids containing 22 to 26 carbon atoms. A common fatty acid in cerebrosides is cerebronic acid.
"Neutral glycosphingolipids" with one disaccharide are termed dihexosides. Tri- and tetra-hexosides are also known. The sugars in these glycosphingolipids are D-glucose, D-galactose, N-acetyl-D-glucosamine and N-acetyl-D-galactosamine. "Neutral glycosphingolipids" are important components of cell surfaces in animal tissues. Their non polar portion is anchored in the double lipid layer of the membrane, while the polar portion projects from the surface.

Another group of sphingolipids is acidic glycosphingolipids, which are termed gangliosides. Their oligosaccharide portion contains one or more sialinic acids. Gangliosides are present in grey matter.

Galactolipids are membrane lipids primarily present in plants. MGDG (monogalactosyldiacylglycerol) and DGDG (digalactosyldiacylglycerol) are the most common galactolipids in higher plants. They are primarily present in plastides and accumulate in particular in the thyalkoids of chloroplasts.

Thus said the embodiment "Cerasome" according to the invention can include the membrane-forming lipids from the group of naturally-derived or synthetic ceramides and naturally-derived or synthetic glycosphingolipids, and more preferably naturally-derived ceramides and naturally-derived glycosphingolipids that are obtained by methods known in the art. In particular naturally-derived ceramides and naturally-derived glycosphingolipids are obtained from botanicals that are rich in ceramides, including but not limited to wheat, by extraction, maceration or percolation of the material with a suitable solvent and, optionally, by partial or complete removal of the solvent. Thus, extracts in accordance with this invention are either so-called solvent-processed fluid extracts or so called dry extracts obtained by evaporation of the whole liquid extract to dryness, e.g. by air drying, spray drying, vacuum oven drying, fluid-bed drying or freeze-drying, and optional washing and/or re-dissolving of this dry extract in at least one suitable solvent. Solvents suitable for extraction, percolation or maceration are known to those experienced in the art. Acetone, chloroform, ethyl acetate, lower alkanols with 1 to 4 carbon atoms, alcohols or a mixture of these and water are particularly suited. Carbon dioxide in fluid or super-critical form and pressurized gases with solvent properties are also suitable as extraction agents.

Within certain embodiments of the invention, "Cerasome" includes membrane-forming lipids comprising ceramides and glycosphingolipids, wherein the amount of ceramides and glycosphingolipids are from about 25 wt% to about 95 wt%, in some cases from about 35 wt% to about 70 wt%, and in other cases from about 40 wt% to about 60 wt%, based on the total weight of all membrane forming lipids.

According to a further embodiment, ceramides may comprise in addition diacylglycosides, wherein the amount of diacylglycosides is from about 20 wt% to about 60 wt%, and in some cases from about 35 wt% to about 45 wt%, based on the total weight of the membrane forming lipids.

### Phospholipids

Within the context of the present invention the term "phospholipid" refers to a lipid or glyceride that contains a phosphate group. Thus the phospholipid may be, for example, lecithin, phosphatidylethanolamine, phosphatidylinositol, phosphatidylserine, diphosphatidyl glycerol (cardiolipin), dilauroyl phosphatidylcholine, dimyristoyl phosphatidylcholine, dipalmitoyl phosphatidylcholine, distearoyl phosphatidylcholine, dioleoyl phosphatidylcholine, dimyristoyl phosphatidylethanolamine, dipalmitoyl phosphatidylethanolamine, dipalmitoyl phosphatidyl glycerol, dimyristoyl phosphatidic acid, dipalmitoyl phosphatide acid, dipalmitoyl phosphatidylserine, dipalmitoyl sphingomyelin, 1-stearoyl-2palmitoyl phosphatidylcholine.

The term "lecithin", within the context of the invention, refers to a naturally occurring or synthetic lecithin, which may be suitably refined. Suitable lecithins include, but are not limited to lecithins derived from soybean, sunflower, or egg. Further suitable lecithins include, but are not limited to dihexanoyl-L-alpha-lecithin, dioctanoyl-L-alpha-lecithin, didecanoyl-L-alpha-lecithin, didodecanoyl-L-alpha-lecithin, ditetradecanoyl-L-alpha-lecithin, dihexadecanoyl-L-alpha-lecithin, dioctadecanoyl-L-alpha-lecithin, dioleoyl-Lalpha-lecithin, dilinoleoyl-L-alpha-lecithin, alpha- palmitoyl. Lecithins are typically mixtures of diglycerides of fatty acids linked to the choline ester of phosphoric acid, and can contain differing amounts of other compounds depending on the method of isolation. Typically, commercial lecithin is a mixture of acetone-insoluble phosphatides. Preferably, the lecithin is obtained from seeds including, but not limited to, soybean, corn, sunflower, rape and lupine, using methods well known in the art.

Soy bean lecithin, obtained as described above, usually comprises phosphatidylcholine, phosphatidylethanolamine and phosphatidic acid as key phospholipid ingredients.

Phospholipids of type PL1 include naturally-derived or synthetic phospholipids selected from among previously described phospholipids, and comprising phosphatidylcholine, phosphatidylethanolamine and phosphatidic acid, wherein the amount of phosphatidylcholine is from about 70 wt% to about 97 wt%, and in some cases from about 73 wt% to about 95 wt%, based on the total weight of the phospholipids, and wherein in some cases the amount of phosphatidylethanolamine is less than about 7 wt%, based on the total weight of the phospholipids, and wherein in some cases the amount of phosphatidic acid is less than about 8 wt%, based on the total weight of the phospholipids.

Phospholipids of type PL2 include naturally-derived or synthetic phospholipids selected from among previously described phospholipids, and comprising phosphatidylcholine, phosphatidylethanolamine and phosphatidic acid, wherein the content of phosphatidylcholine is from about 10 wt% to about 60 wt%, and in some cases from about 15 wt% to about 45 wt%, and in yet other cases from about 20 wt% to about 35 wt%, based on the total weight of the phospholipids.

### Solvents

Embodiments according to the invention include water and solvents from, but not limited to, the group of naturally-derived or synthetic alcohols, naturally-derived or synthetic glycols and naturally-derived or synthetic polyols, including triols, wherein alcohols comprise primary, secondary and tertiary alcohols that are commonly used and accepted in cosmetic formulations and compositions. Alcohols and glycols accepted in cosmetic products include, but are not limited to, ethanol, 1,2-propanediol, 1,3-propanediol, 1,3-butylene glycol, and polyols accepted in cosmetic products include, but are not limited to, glycerol.

According to further embodiments according to the invention the solvents comprising naturally-derived or synthetic alcohols may be combined with naturally-derived or synthetic glycols and/or naturally-derived or synthetic polyols in weight ratios (alcohol: glycol and/or polyol) from about 1 : 0.1 to about 1 : 10, in some cases from about 1 : 0.4 to about 1 : 6, and other cases from about 1 : 0.6 to about 1 : 4, and in yet other cases from about 1 : 1 to about 1 : 2.

### Other Functional Ingredients

Optionally in certain embodiments of the invention the composition according to the invention comprises one or more Other Functional Ingredients that are commonly used in cosmetic formulations and compositions. The term Other Functional Ingredients in connection with the present invention includes substances that may provide benefit to the physical properties of the embodiments and their stability and/or prevent or retard microbial growth. Other Functional Ingredients comprise, without limitation, chelating agents, preservatives, pH buffers, and Other Additives or Actives (as described below, where many of these same ingredients can also be added to personal care products containing Lupine-Based Delivery Systems). The amount of Other Functional Ingredients typically ranges from about 0.1 wt% to about 25 wt%, in some cases about 0.1 wt% to about 8 wt%, and in other cases about 0.1 wt% to about 2 wt%.

### Chelating Agents

Optionally in certain embodiments of the invention the composition according to the invention comprises at least one chelating agent. According to further embodiments of the foregoing, the at least one preservative is selected from but not limited to the group consisting of ethylenediamintetraacetic acid (EDTA) and its salts e.g. disodium EDTA, nitrilotriacetic acid (NTA) and its salts, cosmetic acceptable phosphates and phosphonates, salts and esters of oxalic acid, tartaric acid, phytic acid, glutamic acid and its salts and preferably N,N- bis(carboxymethyl)glutamic acid and its salts and their blends. In general, the chelating agents may be used in any amount which is effective to prevent cationic destabilization of the compositions.

According to a certain embodiment of the invention, the composition comprises tetrasodium N,N-bis(carboxylatomethyl)-L-glutamate in an amount from about 0.001 wt% to about 1 wt%, in some cases from about 0.01 wt% to about 0.5 wt%, and in other cases from about 0.05 wt% to about 0.3 wt%, based on the total weight of the composition.

### Preservatives

Optionally in certain embodiments of the invention the composition according to the invention comprises at least one preservative. According to further embodiments of the foregoing, the at least one preservative is selected from but not limited to the group consisting of butylparaben; ethylparaben; Isobutylparaben, imidazolidinyl urea; methylparaben , sorbic acid and its salts, O- phenylphenol; propylparaben; quaternium-14; quaternium- 5; sodium dehydroacetate; phenoxyethanol, phenoxyisopropanol, benzyl alcohol, polyaminopropyl biguanide, triethylene glycol, piroctone olamine, benzoic acid and its salts, dehydroacetic acid and its salts, diazolidinyl urea, iodopropynyl butylcarbamate, methylisothiazolinone, glyceryl caprylate, phenylethylalcohol, caprylyl glycol, capryloyl glycine, phenylpropanol, ethylhexylglycerin, ethylpropanediol, and their salts and their blends. In general, the preservatives may be used in any amount which is effective to prevent or retard microbial growth and typically about 0.01 wt% to about 5 wt%, and in some cases from about 0.05 wt% to about 3 wt%, based on the total weight of the composition.

According to a further embodiment of the invention, the composition comprises benzyl alcohol and benzoic acid and dehydroacetic acid, wherein the amount of benzyl alcohol is from about 0.01 wt% to about 3 wt%, in some cases from about 0.1 wt% to about 2 wt%, and in other cases from about 0.5 to about 1.0 wt%, and wherein the amount of benzoic acid is from about 0.0015 wt% to about 0.45 wt%, in some cases from about 0.015 wt% to about 0.3 wt%, and in other cases from about 0.075 wt% to about 0.15 wt%, and wherein the amount of dehydroacetic acid is from about 0.001 wt% to about 0.3 wt%, in some cases from about 0.01 wt% to about 0.2 wt%, and in other cases from about 0.05 wt% to about 0.1 wt%, all based on the total weight of the composition.

In one embodiment, the Lupine-Based Delivery System uses a "Cerasome" Delivery System comprising one or more naturally-derived or synthetic ceramides and/or naturally-derived or synthetic glycosphingolipids in an amount from about 0.5 wt% to about 8 wt%, and typically from about 1 wt% to about 4 wt%, a Lupine Powder in an amount from about 0.5 wt% to about 10 wt%, and typically from about 1 wt% to about 4 wt%, at least one alcohol, glycol or triol in an amount from about 5 wt% to about 30 wt%, and water in an amount at least 50 wt%, all based on the total weight of the composition, and wherein the lupine powder comprises protein in an amount from about 35 wt% to about 60 wt%, polysaccharides in an amount from about 7 wt% to about 15 wt%, lipids in an amount from about 7 wt% to about 15 wt%, and fibers in an amount from about 5 wt% to about 35 wt%, all based on the total weight of the "Lupine Powder". In one aspect of this embodiment, the solvent is ethanol in an amount from about 8 wt% to about 25 wt%. In one aspect of this embodiment, the composition also comprises the Other Functional Ingredients of benzyl alcohol, benzoic acid, dehydroacetic acid and salts of N,N-bis(carboxymethyl)glutamic acid.

In another embodiment, the Lupine-Based Delivery System uses a "Liposome I" Delivery System comprising one or more naturally-derived or synthetic phospholipids of type PL2 in an amount from about 1 wt% to about 15 wt%, and typically from about 5 wt% to about 10 wt%, a Lupine Powder in an amount from about 0.5 wt% to about 10 wt%, and typically from about 1 wt% to about 4 wt%, at least one alcohol, glycol or triol, in an amount from about 5 wt% to about 50 wt%, and typically from about 8 wt% to about 35 wt%, and water in an amount at least 50 wt%, and typically at least 60 wt%, all based on the total weight of the composition, and wherein the lupine powder comprises protein in an amount from about 35 wt% to about 60 wt%, polysaccharides in an amount from about 7 wt% to about 15 wt%, lipids in an amount from about 7 wt% to about 15 wt%, and fibers in an amount from about 5 wt% to about 35 wt%, all based on the total weight of the "Lupine Powder", and wherein the phosphatidylcholine content of the phospholipids is from about 15 wt% to about 45 wt%, and in some cases from about 20 wt% to about 35 wt%, based on the total weight of the phospholipids. In one aspect of this embodiment, the solvent comprises glycerol in amount from about 15 wt% to about 50 wt%, and typically from about 15 wt% to about 35 wt%, based on the total weight of the composition. In another aspect of this embodiment, the solvent comprises 1,3-butylene glycol in an amount from about 10 wt% to about 25 wt%, based on the total weight of the composition. In yet another aspect of this embodiment, the solvent comprises 1,2-propanediol or 1,3-propanediol in an amount from about 15 wt% to about 25 wt%, based on the total weight of the composition. In still another aspect of this embodiment, the solvent comprises ethanol in an amount from about 5 wt% to about 15 wt%, based on the total weight of the composition. In a further aspect of this embodiment, the composition of any previously presented aspect of this embodiment also comprises the Other Functional Ingredients of benzyl alcohol, benzoic acid, dehydroacetic acid and salts of N,N-bis(carboxymethyl)glutamic acid.

In another embodiment, the Lupine-Based Delivery System uses a "Liposome II" Delivery System comprising one or more naturally-derived or synthetic phospholipids of type PL1 in an amount from about 1 wt% to about 25 wt%, and typically from about 5 wt% to about 15 wt%, a Lupine Powder in an amount from about 0.5 wt% to about 10 wt%, and typically from about 1 wt% to about 4 wt%, at least one alcohol, glycol or triol, in an amount from about 5 wt% to about 50 wt%, and typically from about 10 wt% to about 40 wt%, and water in an amount at least 50 wt%, and typically at least 60 wt%, all based on the total weight of the composition, and wherein the lupine powder comprises protein in an amount from about 35 wt% to about 60 wt%, polysaccharides in an amount from about 7 wt% to about 15 wt%, lipids in an amount from about 7 wt% to about 15 wt%, and fibers in an amount from about 5 wt% to about 35 wt%, all based on the total weight of the "Lupine Powder". In one aspect of this embodiment, the solvent comprises glycerol, typically from about 5 wt% to about 25 wt%, based on the total weight of the composition. In another aspect of this embodiment, the solvent comprises 1,3-butylene glycol, typically from about 5 wt% to about 25 wt%, based on the total weight of the composition. In yet another aspect of this embodiment, the solvent comprises ethanol, typically from about 5 wt% to about 25 wt%, based on the total weight of the composition. In a further aspect of this embodiment, the composition of any previously presented aspect of this embodiment also comprises the Other Functional Ingredients of benzyl alcohol, benzoic acid, dehydroacetic acid and salts of N,N-bis(carboxymethyl)glutamic acid.

Testing of physical stability can be performed over a range of temperatures, and using various criteria, that are of specific interest to one skilled in the art. For Lupine-Based Delivery Systems, Personal Care products, and Cosmetic products, including ingredients and compositions, testing of physical stability for purposes of this invention is assessed at temperatures ranging from about -10 °C to about 60 °C. For purposes of this invention, physical stability of exemplary Lupine-Based Delivery System compositions was measured on a weekly basis by visual observation of samples (approximately 25 grams of sample contained in a closed, clear, 30 ml bottle constructed of polyethylene terephthalate) that are stored at 40 °C or 50 °C under atmospheric conditions within a Heraeus UT12 oven. An exemplary composition was deemed to have failed in physical stability if two distinct and separated layers were observed at the time of measurement. For purposes of this invention, an exemplary composition was considered to have physical stability or be "physically stable" if the composition had not failed in physical stability after 30 days at 40 °C or 15 days at 50 °C.

### Personal care products and exemplary formulations using Lupine-Based Delivery Systems

The Lupine-Based Delivery System compositions disclosed herein may be used as an ingredient in creating a Cosmetic or Personal Care product that provides increased effectiveness for topical application with a moisturizing effect, a barrier effect on the skin, sensory benefits, and as a texturing agent, with such benefits provided solely by the Lupine-Based Delivery System or in combination with other cosmetic ingredients. A key aspect includes a cosmetic composition that has a Lupine-Based Delivery System composition as a first moisturizing ingredient on its own or with combination of other moisturizing ingredients or actives. Lupine-Based Delivery System compositions may also provide delivery enhancement and increase the efficacy of other actives, whether the actives are a component of the Lupine-Based Delivery System or an ingredient of the personal care product. The Lupine-Based Delivery System composition is generally present at about 0.1 to about 20% by weight in such products, in some cases between about 0.5 and about 15% by weight, and in still other cases is present between about 1% and about 10% by weight. The composition can be used with a wide range of personal care products having a base media that can comprise at least one member selected from the group consisting of cosmetic oil (i.e. oils compatible for cosmetic uses), water, alcohol and combinations thereof, all by way of example only.

The Lupine-Based Delivery System composition can be added to Personal Care products as is, or as dispersion in water or any suitable organic solvent or combination of solvents and/or water.

The Cosmetic composition may include, in accordance with one exemplary embodiment, Lupine-Based Delivery System composition that when applied to the skin can provide moisturizing benefits. The Cosmetic composition containing a Lupine-Based Delivery System may minimize itching of the skin, and may assist in delivering other cosmetic active effectively to the skin. Additional components may be included in the cosmetic composition in order to achieve various beneficial results when applied to the body or hair of a user. All of the components of the cosmetic composition might be natural components or can be combination of natural and synthetic components. The cosmetic composition can be provided with a number of different components in varying quantities in order to achieve a desired result. By way of example, the cosmetic composition may be provided for use on dry or normal skin, and can be applied to the skin of a user in order to increase skin moisture, look, and/or softness.

The Cosmetic composition can also be used to provide moisturization to hair and improve hair luster, hair strength. Lupine-Based Delivery System composition may also act as deeper skin moisturizer by maintaining moisture in the skin by creating a barrier system on the skin surface, and in some embodiments may also impart moisture to deeper layers of the skin. Lupine-Based Delivery System composition may provide high hydration to skin by easily absorbing, and might accelerate a natural fresh look to the skin. Lupine-Based Delivery System composition may quickly absorb into the skin in a few minute or less, such that it is no longer on the skin surface, and able to provide moisturizing and delivery of active benefits to the deeper skin layers of the epidermis.

Exemplary Cosmetic, toiletry and topical health care type personal care products in which the Lupine-Based Delivery System composition may be employed include, without limitation, leave-on personal care products (i.e., products that are left on keratinous substrates after application); rinse-off personal care products (i.e., products that are washed or rinsed from keratinous substrates during or within a few minutes of application); at least one of shampoos; chemical and non-chemical hair curling and hair straightening products; hair style maintaining and hair conditioning products; lotions and creams for nails, hands, feet, face, scalp and/or body; hair dye; face and body makeup; nail care products; astringents; deodorants; antiperspirants; antiacne; antiaging; depilatories; colognes and perfumes; skin protective creams and lotions (such as sunscreens); skin and body cleansers; skin conditioners; skin toners; skin firming compositions; skin tanning and lightening compositions; liquid soaps; bar soaps; bath products; shaving products; and oral hygiene products (such as toothpastes, oral suspensions, and mouth care products), any or the foregoing of which may additionally contain pharmaceutical, phytopharmaceutical and/or neutraceutical ingredients.

The form of such Personal Care products includes, without limitation, a liquid, gel, spray, emulsion (such as lotions and creams), shampoo, pomade, foam, tablet, stick (such as lip care products), makeup, suppositories, among others, any of which can be applied to the skin or hair and which typically are designed to remain in contact there with until removed, such as by rinsing with water or washing with shampoo or soap. Other forms could be gels that can be soft, stiff, or squeezable. Emulsions can be oil-in-water, water-in-oil, water-and-silicone, or multiphase. Sprays can be non-pressurized aerosols delivered from manually pumped finger-actuated sprayers or can be pressurized aerosols. In some embodiments, exemplary Lupine-Based Delivery System composition are formulated in aerosol compositions such as mousse, spray, or foam forming formulation, where a chemical or gaseous propellant is used.

Personal Care products containing a Lupine-Based Delivery System , can be broadly categorized as gel or emulsions products, water-in-oil emulsions, oil-in-water emulsions, or aqueous or alcohol based systems.

Lupine-Based Delivery System composition can be effective with a broad range of cosmetic oils. In general, any natural or synthetic oil for cosmetic use is suitable for the composition of present invention. Non-limiting examples of such as esters (e.g., alkyl benzoates having between 12 to 15 carbons), triglycerides (e.g., Caprylic/Caprylate triglyceride), hydrocarbons (e.g., mineral oil, Petroleum Jelly), natural oils (e.g., Jojoba Oil, Safflower Oil, Olive Oil, Sweet Almond Oil, Mango Butter, Sunflower Oil, Aloe Butter, Cocoa Butter, Shea Butter, Palm Kernel Flakes, Beeswax, Lanolin Butter, Sweet Orange Oil, Lemon Oil, Avocado oil, Coconut Oil, Palm Oil, Sesame Oil, Peanut Oil, Peach Kernel Oil, Wheat Germ Oil, Macadamia Nut Oil, Night Primrose oil, Soya Oil and the derivatives thereof.), and castor oil, among others. Suitable oils are also disclosed, for example, at column 3, line 37, to column 4, line 4, of U.S. Patent No. 5,736,125; hereby incorporated by reference. Silicone oils may also be used as cosmetic oils.

Suitable synthetic oils are at least one member selected from the group consisting of fatty alcohols; fatty acid esters such as isopropyl myristate, palmitate, stearate and isostearate; oleyl oleate; isocetyl stearate; hexyl laurate; dibutyl adipate; dioctyl adiphate; myristyl myristate; oleyl erucate; polyethylene glycol and it derivatives; polyglyceryl fatty acid esters; and cetyl palmitate, by way of example only.

Silicone oils are also suitable. Useful silicone oils are non-volatile silicone oils known by INCI names that include dimethicone or dimethiconol. Volatile silicone oils such as cyclomethicones may also be used.

Water-in-oil emulsions can be prepared by mixing together (1) a heated (i.e., melted) solution of the oil phase and (2) an aqueous phase, the aqueous phase being at a temperature similar to the oil (typically within about 10 °C); and then cooling the mixture while stirring. While bringing the temperature of emulsion down Lupine-Based Delivery System composition could be added below 45C as emulsion been emulsified and in process of adding other temperature sensitive cosmetic ingredients like preservative, actives. Alternatively, the Lupine-Based Delivery System composition could be added to the aqueous phase if formulation is made without any heating like cold process formulations. Lupine-Based Delivery System composition is generally added below 45 °C to room temperature in any cosmetic formulation. Regardless of the manner in which the Lupine-Based Delivery System composition is added, the ratio of the aqueous phase to the oil phase can be, for example, about 0.5:1 to about 9:1.

The following are non-limiting examples of Cosmetic formulations comprising water-in-oil emulsions:
A) Skin Moisturizer
   Water - about 50 to about 90 wt%
   Silicone - about 1 to about 10 wt%
   Emulsifier - about 0.5 to about 5 wt%
   Emollient - about 5 to about 20 wt%
   Lupine-Based Delivery System composition - about 0.1 to about 20 wt%
   Polymer - about 0.1 to about 5 wt%
   Other Additives or Actives - about 0.1 to about 3 wt%
B) Sunscreen
   Water - about 50 to about 90 wt%
   Silicone - about 1 to about 10 wt%
   Emulsifier - about 0.5 to about 5 wt%
   Emollient - about 5 to about 20 wt%
   Lupine-Based Delivery System composition - about 0.1 to about 20 wt%
   Polymer - about 0.1 to about 5 wt%
   Sunscreen Active - about 1 to about 25 wt%
   Other Additives or Actives - about 0.1 to about 3 wt%
C) Antiperspirant Deodorant
   Water - about 50 to about 90 wt%
   Silicone - up to about 10 wt%
   Emulsifier - about 0.5 to about 5 wt%
   Ethanol or other solvents - about 0 to about 20 wt%
   Lupine-Based Delivery System composition - about 0.1 to about 20 wt%
   Polymer - up to about 5 wt%
   APDO actives - about 0.1 to about 30 wt%
   Other Additives or Actives - about 0.1 to about 5 wt%

Oil-in-water emulsions are prepared by mixing together (1) a heated (i.e., melted) solution of the oil phase and (2) an aqueous phase, the aqueous phase being at a temperature similar to the emollient solution (typically within about 10 °C); and then cooling the mixture while stirring. As with the water-in-oil emulsions, the Lupine-Based Delivery System composition may initially be added to the aqueous phase if formulation is cold processable or added post-emulsification below 45 °C. The ratio of the oil phase to the water phase can be, for example, about 0.1:1 to about 1:1. The following are non-limiting examples of cosmetic formulations comprising oil-in-water emulsions:
A) Skin Moisturizer
   Water - about 50 to about 90 wt%
   Emulsifier - about 0.5 to about 5 wt%
   Emollient - about 1 to about 20 wt%
   Lupine-Based Delivery System composition - about 0.1 to about 20 wt%
   Polymer - about 0.1 to about 5 wt%
   Other Additives or Actives - about 0.1 to about 3 wt%
B) Sunscreen
   Water - about 50 to about 90 wt%
   Emulsifier - about 0.5 to about 5 wt%
   Emollient - about 1 to about 20 wt%
   Ethanol and/or other organic solvent - up to about 35 wt%
   Lupine-Based Delivery System composition - about 0.1 to about 20 wt%
   Polymer - about 0.1 to about 5 wt%
   Sunscreen Active - about 1 to about 25 wt%
   Other Additives or Actives - about 0.1 to about 3 wt%
C) Mousse or other hair styling product
   Water - about 50 to about 90 wt%
   Emulsifier - about 0.5 to about 1 wt%
   Surfactant - about 0.1 to about 2 wt%
   Lupine-Based Delivery System composition - about 0.1 to about 20 wt%
   Polymer - up to about 5 wt%
   Other Additives or Actives - about 0.1 to about 2 wt%
   Solvent - about 1 to about 25 wt%
   Propellant - up to about 10 wt%
D) Antiperspirant Deodorant
   Water - about 50 to about 90 wt%
   Silicone - up to about 10 wt%
   Emulsifier - about 0.5 to about 5 wt%
   Ethanol or other solvents - about 1 to about 35 wt%
   Lupine-Based Delivery System composition - about 0.1 to about 20 wt%
   Polymer - up to about 5 wt%
   APDO actives - about 0.1 to about 30 wt%
   Other Additives or Actives - about 0.1 to about 5 wt%

The following are non-limiting examples of cosmetic formulations comprising alcohol or aqueous systems.
A) Coloring Shampoo
   Water - about 50 to about 90 wt %
   Surfactant - about 2 to about 20 wt%
   Foam booster -up to about 20 wt%
   Lupine-Based Delivery System composition - about 0.1 to about 20 wt%
   Polymer - up to about 5 wt%
   Other Additives or Actives - about 0.1 to about 10 wt%
B) Hair Spray (aerosol and non-aerosol)
   Water - about 10 to about 90 wt%
   Lupine-Based Delivery System composition - about 0.1 to about 20 wt%
   Polymer - up to about 5 wt%
   Ethanol or other solvents - about 10 to about 90 wt%
   Optional Propellant for an aerosol - up to about 50 wt%
   Other Additives or Actives - about 0.1 to about 2 wt%
C) Shampoo
   Water - about 50 to about 90 wt%
   Surfactant - up to about 20 wt%
   Foam booster - about 2 to about 20 wt%
   Lupine-Based Delivery System composition - about 0.1 to about 20 wt%
   Polymer - up to about 5 wt%
   Other Additives or Actives - about 0.1 to about 10 wt%
D) Hair Styling products
   Water - about 10 to about 90 wt%
   Lupine-Based Delivery System composition - about 0.1 to about 20 wt%
   Polymer - about 0.1 to about 5 wt%
   Ethanol or other solvents- up to about 10 wt%
   Other Additives or Actives - about 0.1 to about 10 wt%
E) Body cleansing products
   Water - about 50 to about 90 wt%
   Surfactant - about 2 to about 20 wt%
   Lupine-Based Delivery System composition - about 0.1 to about 20 wt%
   Polymer - up to about 5 wt%
   Foam booster - up to about 20 wt%
   Other Additives or Actives - about 0.1 to about 10 wt%.

In products in which emollients are employed, any suitable emollient for use in cosmetic compositions can be used. Examples of suitable emollients include at least one of esters (e.g., C12-15 alkyl benzoate) and triglycerides (e.g., Caprylic/caprylate triglyceride); hydrocarbon oils (e.g., mineral oil), natural oil (e.g., Jojoba oil, safflower oil), tridecyl trimellitate, sunflower oil, castor oil, among other compounds used to impart desired or improved sensory or aesthetic properties of a personal care composition. Many emollients used in cosmetic compositions also fall within the category of cosmetic oils.

In products in which emulsifiers are employed, any suitable cosmetic emulsifier having a hydrophilic-lipophilic balance (HLB) in the range of about 1 to about 20 can be used. The emulsifier can be nonionic, cationic, anionic, or amphoteric or a combination of such emulsifiers can be used.

Examples of nonionic emulsifiers are at least one of laureths, e.g. laureth-4; ceteths, e.g. ceteths-1; polyethylene glycol cetyl ether; Cetearth, e.g. cetearth-25; polyglycol fatty acid glycerides; hydroxylated lecithin; lactyl esters of fatty acids; and alkyl polyglycosides.

Examples of cationic emulsifiers are at least one of cetyldimethyl-2-hydroxyethylammonium dihydrogenphosphate; cetyltrimonium chloride; cetyltrimonium bromide; cocotrimonium methosulfate; as well as emulsifiers that contain quaternized nitrogen.

Anionic emulsifiers include, for example, at least one of alkyl sulfates; alkyl ether sulfates; alkylsulfonates; alkylarylsulfonates; alkyl succinates; alkyl sulfosuccinates; N-alkylsarcosinates; acyl taurates; acyl isethionates; alkyl phosphates; alkyl ether phosphates; alkyl ether carboxylates; alpha-olefinsulfonates, and the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Surfactants and/or foam boosters may also be employed and like the emulsifiers can be nonionic, cationic, anionic, or amphoteric or a combination of such surfactants can be used.

Suitable anionic surfactants are for example, at least one of alkyl sulfates; alkyl ether sulfates; alkylsulfonates; alkylarylsulfonates; alkyl succinates; N-alkylsarcosinates; acyl taurates; acyl isethionates; alkyl phosphates; alkyl ether phosphates; alkyl ether carboxylates; alpha olefinsulfonates, and may include the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Suitable amphoteric surfactants are, for example, at least one of alkylbetaines; alkylamidopropylbetaines; alkylsulfobetaines; alkyl glycinates; alkylcarboxyglycinates; alkyl amphoacetates or amphopropionates; and alkyl amphodiacetates or amphodipropionates. For example, it is possible to use cocodimethylsulfopropylbetaine, laurylbetaine, and cocamidopropylbetaine or sodium cocamphopropionate as surfactants.

Examples of nonionic surfactants are the reaction products of aliphatic alcohols having between 6 and 20 carbon atoms in the alkyl chains, which can be linear or branched with ethylene oxide and/or propylene oxide. Also suitable are at least one of alkylamine oxides; mono- or dialkylalkanolamides; fatty acid esters of polyethylene glycols; alkyl polyglycosides and sorbitan ether esters.

Examples of cationic surfactant are quaternary ammonium compounds, for example, cetyltrimethylammonium chloride, as well as other surfactants that contain a quaternized nitrogen.

In personal care compositions in which polymers are used with the Lupine-Based Delivery System, any one or more polymer(s) can be of any type, including those which are at least one of nonionic, amphoteric or zwitterionic, anionic, cationic or mixtures of such types of polymers.

Exemplary synthetic polymers include at least one of vinyl pyrrolidone or acrylate homopolymer and copolymers, including carbomer, and those which have a vinyl acetate group or acrylate or acrylamide or taurate group. Natural non-ionic polymers suitable for the composition of the present invention can comprise at least one of cellulose, starches, chitosan, xanthan gum, guar gum, neutralized shellac and their derivatives. Cationic derivatives or chemical modified forms of natural polymers may also be included, such as chemically modifies starches, celluloses, guar and xanthan gum e.g., methylcellulose, hydroxyethylcellulose, quaternized guar.

The polymer may also include silicone compounds, such as, for example, at least one of polydiorganosiloxanes, polydialkylsiloxanes, polyalkylsiloxanes, polyarylsiloxane, silicone resins, silicone gums or dimethicone copolyols and aminofunctional silicone compounds such as amodimethicone. Other silicone compounds include graft polymers of organosiloxane and polyethyloxazolines compounds known with the INCI name Polysilicone-9. Any polymeric compound having an INCI name including silicone, methicone, dimethicone, or siloxane as part of its name may be used.

In products in which propellant or solvents are employed, those may include at least one of isobutane, butane, dimethyl ether, and ethanol, among others.

Other Additives or Actives include one or more of additive compounds, active compounds, and moisturizing and humectants compounds. Examples of other additive compounds include one or more members selected from the group consisting of silicone based plasticizers, natural or synthetic compounds (e.g., polysaccharides, natural or synthetic gums, stabilizers, anionic and nonionic associative thickener or rheology modifiers soluble in oil or water phase), among other compounds. The additives may include at least one compound selected from the group consisting of preservatives, stabilizers (e.g., Xanthan Gum), antioxidant (e.g., Vitamins), rheology modifiers, fragrances, and pigments, among other additives.

In some Personal Care products, active compounds that interact with or protect skin or hair can be included. Examples of such active compounds include at least one of sunscreen compounds (e.g. zinc oxide, titanium dioxide, octinoxate, octocrylene, ethylhexyl salicylate, oxybenzone); skin whiteners (e.g. salicylic acid); anti-cellulite compounds; anti-aging compounds (e.g., polypeptides such as Argininie/Lysine, Argininie PCA, Aspergillus/Aspidosperma Quebracho Ferment, botanical actives, Bifida Ferment Lysate, Calophylum Inorhylum seed oil, camellia sinensis extract, ceramides, chlorella vulgaris extract, coriolus versicolor extract, corylus avellana (hazel) seed extract, erythorbic acid, hydrolyzed elastins, hydrolyzed proteins, hydrolyzed soy flour, hydrolyzed peptides, and Vitamins A, E, C, K, and B5 as well as Niacinamide); antidandruff compounds (e.g., zinc pyrithione); APDO compounds (e.g., aluminum chlorohydrate, aluminum zirconium tetra chlorohydrex); vitamins (e.g., Tocopherol natural, synthetic Tocopherol, synthetic tocopheryl acetate, Retinol, Retinyl palmitate, Retinyl acetate, Provitamin B-5, ascorbic acid, sodium ascorbyl phosphate, Ascorbyl glucoside, Magnesium ascorbyl phosphate); insect repellents; and Coenzymes and Enzymes (e.g., Ubiquinone, Coenzyme Q10), all by way of example only. Botanicals (e.g., Aloe vera, Green tea extract, Grape seed extract, Isoflavones, Chamomille/bisabolol, Fennel, Ginko, Ginseng, Guava); Alpha Hydroxy Acids (e.g., Citric acid, Glycolic acid, Lactic acid); Sugar cane extracts; Avena Sativa (Oat) Kernel Protein, and Avocado Sterols, proteins, peptides, copper peptides, fermented biopolymers, betaglucan, Hyaluronic acid and its derivatives like sodium hyluronate. It will be appreciated that certain active compounds may fall into more than one category and/or be used to accomplish more than one result.

In Personal Care products, moisturizing and Humectants compounds that interact with or skin or hair could be simple or complex mixtures of chemical agents specially designed to make the external or upper layers of the skin (epidermis) softer and more pliable. They increase the skin's hydration or water content by reducing evaporation or by bringing moisture to the skin. Types of moisturizers and Humectants include, for example, naturally occurring skin lipids and sterols, as well as artificial or natural oils, emollients, lubricants. Examples of such moisturizing or humectants compounds include glycol (e.g., glycerin, propylene glycol, dipropylene glycol, butylene glycol MP diol, sorbitol, and hexylene glycol), Polysaccharides (e.g., Hyaluronic acid and its salts, B-1, 3-glucans, Chitosan), Botanicals (e.g., Aloe vera, Ginkgo, green tea extract, rose extract, sugar extract). Natural oils (e.g., coconut oil, grape seed oil, jojoba oil, olive oil, Argan oil), polyethylene glycol ether of methyl glucose (e.g., Methyl Gluceth-10), green algae, natural and herbal extract design for moisturization like alpha lipoic acid, peptides, proteins amino acids and collagens and betaine among others.

Personal Care products may employ the Lupine-Based Delivery System composition alone as a moisturizing effect and as a barrier affect on the skin good sensory attributes, and/or as another aspect of Lupine-Based Delivery System in a cosmetic composition that further has a texturing agent in combination with other cosmetic ingredients. The Lupine-Based Delivery System composition provides high hydration to skin by easily absorbing into the skin and enhancing a natural fresh look.

The following Examples are provided to illustrate certain embodiments of the invention and do not limit the scope of the appended claims.

### Example 1. Preparation of a "Cerasome" with Lupine Extract

| Ingredient | Amount |
|---|---|
| Deionized Water | 157.0 g |
| Ethanol | 33.0 g |
| Glycosphingolipids** | 6.0 g |
| Lupinus Albus Seed Extract* | 4.0 g |

| | |
|---|---|
| * a Lupine Powder comprised of 38 wt% proteins, 10 wt% polysaccharides, 14 wt% lipids, 28 wt% fibers ** vegetable Ceramides comprising 50 to 55 wt% glycosphingolipids | |

Lupinus Albus Seed Extract was added to the water while stirring at 350 to 450 rpm and the mixture was heated at a rate of approximately 2 °C per minute to 80 ± 2 °C. The temperature and stirring were maintained for 30 minutes. Following this step the water and lupine mixture was cooled to room temperature. In a separate container, Glycosphingolipids were added to the ethanol and the mixture was heated to 50 ± 2 °C while stirring at 50 to 100 rpm. Stirring and temperature were maintained for 15 min until the Glycosphingolipids were fully dissolved. The Glycosphingolipids solution was slowly added to the water and lupine mixture while homogenizing by means of an IKA T25 digital Ultra-Turrax at 14,000 rpm. Homogenization was continued for 5 minutes at ambient conditions. The particle size, as measured by Photon Correlation Spectroscopy as described previously, was 234 nm. The composition was physically stable, with physical stability at 40 °C greater than 168 days as measured in accordance with the previously described method.

### Examples 2 - 20. Preparations of "Liposome I" with Lupine Extract

Preparations of Examples 2 - 20 were prepared as follows. Lupinus Albus Seed Extract was added to the water while stirring at 350 to 450 rpm and the mixture was heated at a rate of approximately 2 °C per minute to 80 ± 2 °C. The temperature and stirring were maintained for 30 minutes. Following this step the water and lupine mixture was cooled to room temperature. Tetrasodium Glutamate Diacetate and 97% of the indicated amount of Phospholipid type PL2 were added to the water and lupine mixture while homogenizing by means of an IKA T25 digital Ultra-Turrax at 14,000 rpm for 2 minutes. In a separate container, the remaining Phospholipid type PL2 was added to the Solvent (one of ethanol, 1,2-propanediol, 1,3-propanediol, 1,3-butylene glycol or glycerol in the amounts listed in the table below) and the mixture was heated to 78 ± 2 °C while stirring at 50 to 100 rpm. Stirring and temperature were maintained for 3-4 hours until the Phospholipid type PL2 was fully dissolved. Subsequently this mixture was cooled to room temperature. The solution of Phospholipid type PL2 and Solvent was slowly added to the aqueous phase while homogenizing at 14,000 rpm. Homogenization was continued for 3 minutes at ambient conditions. The preservative (79 wt% benzyl alcohol, 12 wt% benzoic acid, 8 wt% dehydroactic acid, Optiphen® BD from Ashland, Inc.) was added while homogenizing at 14,000 rpm. Homogenization was continued for an 2 additional minutes at ambient conditions. The particle size was measured by Photon Correlation Spectroscopy as described previously, and physical stability was measured in accordance with previously described methods.

| | Example 2 | Example 3 | Example 4 | Example 5 | Example 6 | Example 7 |
|---|---|---|---|---|---|---|
| Deionized Water (g) | 140.4 | 120.4 | 100.4 | 80.4 | 160.4 | 140.4 |
| Ethanol (g) | | | | | | |
| 1,2-Propanediol (g) | | | | | | |
| 1,3-Propanediol (g) | | | | | | |
| 1,3-Butylene glycol (g) | | | | | 20.0 | 40.0 |
| Glycerol (g) | 40.0 | 60.0 | 80.0 | 100.0 | | |
| Tetrasodium Glutamate Diacetate (g) | 0.2 | 0.2 | 0.2 | 0.2 | 0.2 | 0.2 |
| Phospholipid Type PL2 (g) | 13.4 | 13.4 | 13.4 | 13.4 | 13.4 | 13.4 |
| Phospholipid Type PL2, wt% of phosphatidylcholine | 25 | 25 | 25 | 25 | 25 | 25 |
| Lupinus Albus Seed Extract (g) | 4.0 | 4.0 | 4.0 | 4.0 | 4.0 | 4.0 |
| Lupinus Albus Seed Extract Composition | (A) | (A) | (A) | (A) | (A) | (A) |
| Preservative* (g) | 2.0 | 2.0 | 2.0 | 2.0 | 2.0 | 2.0 |
| Particle size, nm | 367 | 344 | 343 | 311 | 296 | 286 |
| Physical Stability | >35† days @ 50 °C | >35† days @ 50 °C | 21 days @ 50 °C | 7 days @ 50 °C | >35† days @ 50 °C | >35† days @ 50 °C |
| Was Example Composition Physically Stable? | Yes | Yes | Yes | No | Yes | Yes |

| | | | | | | |
|---|---|---|---|---|---|---|
| (A) a Lupine Powder comprised of 38 wt% proteins, 10 wt% polysaccharides, 14 wt% lipids, 28 wt% fibers * Preservative comprised 79 wt% benzyl alcohol, 12 wt% benzoic acid, 8 wt% dehydroacetic acid † Test was stopped at the indicated number of days, with the sample still stable | | | | | | |

| | Example 8 | Example 9 | Example 10 | Example 11 | Example 12 | Example 13 | Example 14 |
|---|---|---|---|---|---|---|---|
| Deionized Water (g) | 120.4 | 140.4 | 120.4 | 100.4 | 140.4 | 120.4 | 160.4 |
| Ethanol (g) | | | | | | | 20.0 |
| 1,2-Propanediol (g) | | 40.0 | 60.0 | 80.0 | | | |
| 1,3-Propanediol (g) | | | | | 40.0 | 60.0 | |
| 1,3-Butylene glycol (g) | 60.0 | | | | | | |
| Glycerol (g) | | | | | | | |
| Tetrasodium Glutamate Diacetate (g) | 0.2 | 0.2 | 0.2 | 0.2 | 0.2 | 0.2 | 0.2 |
| Phospholipid Type PL2 (g) | 13.4 | 13.4 | 13.4 | 13.4 | 13.4 | 13.4 | 13.4 |
| Phospholipid Type PL2, wt% of phosphatidylcholine | 25 | 25 | 25 | 25 | 25 | 25 | 25 |
| Lupinus Albus Seed Extract (g) | 4.0 | 4.0 | 4.0 | 4.0 | 4.0 | 4.0 | 4.0 |
| Lupinus Albus Seed Extract Composition | (A) | (A) | (A) | (A) | (A) | (A) | (A) |
| Preservative* (g) | 2.0 | 2.0 | 2.0 | 2.0 | 2.0 | 2.0 | 2.0 |
| Particle size, nm | 407 | 284 | 260 | 278 | 311 | 380 | 295 |
| Physical Stability | <7 days @ 50 °C | >35† days @ 50 °C | >35† days @ 50 °C | 7 days @ 50 °C | >35† days @ 50 °C | 7 days @ 50 °C | >35† days @ 50 °C |
| Was Example Composition Physically Stable? | No | Yes | Yes | No | Yes | No | Yes |

| | | | | | | | |
|---|---|---|---|---|---|---|---|
| (A) a Lupine Powder comprised of 38 wt% proteins, 10 wt% polysaccharides, 14 wt% lipids, 28 wt% fibers * Preservative comprised 79 wt% benzyl alcohol, 12 wt% benzoic acid, 8 wt% dehydroacetic acid † Test was stopped at the indicated number of days, with the sample still stable | | | | | | | |

| | Example 15 | Example 16 | Example 17 | Example 18 | Example 19 | Example 20 |
|---|---|---|---|---|---|---|
| Deionized Water (g) | 140.4 | 120.4 | 160.4 | 160.4 | 160.4 | 100.4 |
| Ethanol (g) | 40.0 | | | | | |
| 1,2-Propanediol (g) | | | | 20.0 | | |
| 1,3-Propanediol (g) | | | | | 20.0 | 80.0 |
| 1,3-Butylene glycol (g) | | | | | | |
| Glycerol (g) | | 60.0 | 20.0 | | | |
| Tetrasodium Glutamate Diacetate (g) | 0.2 | 0.2 | 0.2 | 0.2 | 0.2 | 0.2 |
| Phospholipid Type PL2 (g) | 13.4 | 13.4 | 13.4 | 13.4 | 13.4 | 13.4 |
| Phospholipid Type PL2, wt% of phosphatidylcholine | 25 | 25 | 25 | 25 | 25 | 25 |
| Lupinus Albus Seed Extract (g) | 4.0 | 4.0 | 4.0 | 4.0 | 4.0 | 4.0 |
| Lupinus Albus Seed Extract Composition | (A) | (B) | (A) | (A) | (A) | (A) |
| Preservative* (g) | 2.0 | 2.0 | 2.0 | 2.0 | 2.0 | 2.0 |
| Particle size, nm | 272 | 311 | ‡ | ‡ | ‡ | ‡ |
| Physical Stability | 14 days @ 50 °C | >168† days @ 40 °C | Unstable ‡ | Unstable ‡ | Unstable ‡ | Unstable ‡ |
| Was Example Composition Physically Stable? | No | Yes | No | No | No | No |

| | | | | | | |
|---|---|---|---|---|---|---|
| (A) a Lupine Powder comprised of 38 wt% proteins, 10 wt% polysaccharides, 14 wt% lipids, 28 wt% fibers (B) a Lupine Powder comprised of 57 wt% proteins, 11 wt% polysaccharides, 13 wt% lipids, 5 wt% fibers * Preservative comprised 79 wt% benzyl alcohol, 12 wt% benzoic acid, 8 wt% dehydroacetic acid † Test was stopped at the indicated number of days, with the sample still stable ‡ Example was unstable at preparation and measurement of particle size was not possible | | | | | | |

### Examples 21- 30. Preparations of "Liposome II" with Lupine Extract

Preparations of Examples 21- 30 were prepared as follows. Lupinus Albus Seed Extract was added to the water while stirring at 200 to 300 rpm and the mixture was heated at a rate of approximately 2 °C per minute to 80 ± 2 °C. The temperature and stirring were maintained for 30 minutes. Following this step the water and lupine mixture was cooled to room temperature and Tetrasodium Glutamate Diacetate was added to the water and lupine mixture while stirring at 200 to 300 rpm for 2 minutes. In a separate container, Phospholipid type PL1 was added to the Solvent (one of ethanol, 1,3-butylene glycol or glycerol in the amounts listed in the table below) at ambient temperature and the mixture was occasionally stirred at 50 to 100 rpm for 45 minutes until the Phospholipid type PL1 was fully dissolved. The solution of Phospholipid type PL1 and Solvent was slowly added to the aqueous phase while homogenizing by means of an IKA T25 digital Ultra-Turrax at 14,000 rpm. Homogenization was continued for 3 minutes at ambient conditions. In Examples 29 and 30, Potassium Dihydrogen Phosphate and Sodium Hydroxide solution was added to 5 grams of water in a separate container, and stirred until fully dissolved to produce a premix, the premix then slowly added to the homogenized batch and stirred at 200 to 300 rpm for 5 minutes. The preservative, if included within the composition, was added while homogenizing at 14,000 rpm, and homogenization was continued for 2 additional minutes at ambient conditions. Where the amount of ethanol in the composition exceeded 10 wt%, preservative was not added as the ethanol provided preservation. Where a combination of ethanol and diol or triol were included in the composition, the ethanol served as Solvent, and the diol or triol was added to the water coincident with the Tetrasodium Glutamate Diacetate. The particle size and physical stability were measured in accordance with the previous Examples.

| | Example 21 | Example 22 | Example 23 | Example 24 | Example 25 |
|---|---|---|---|---|---|
| Deionized Water (g) | 66.9 | 71.4 | 61.4 | 51.4 | 51.4 |
| Ethanol (g) | | 16.5 | 16.5 | 16.5 | 16.5 |
| 1,3-Butylene glycol (g) | 20.0 | | 10.0 | 20.0 | |
| Glycerol (g) | | | | | 20.0 |
| Tetrasodium Glutamate Diacetate (g) | 0.1 | 0.1 | 0.1 | 0.1 | 0.1 |
| Phospholipid Type PL1 (g) | 10.0 | 10.0 | 10.0 | 10.0 | 10.0 |
| Phospholipid Type PL1, wt% of phosphatidylcholine | 92 | 92 | 92 | 92 | 92 |
| Lupinus Albus Seed Extract (g) | 2.0 | 2.0 | 2.0 | 2.0 | 2.0 |
| Lupinus Albus Seed Extract Composition | (A) | (A) | (A) | (A) | (A) |
| Preservative* (g) | 1.0 | 0 | 0 | 0 | 0 |
| Particle size, nm | 312 | 242 | 217 | 192 | 179 |
| Physical Stability | >18† days @ 50 °C | >18† days @ 50 °C | >18† days @ 50 °C | >18† days @ 50 °C | >18† days @ 50 °C |
| Was Example Composition Physically Stable? | Yes | Yes | Yes | Yes | Yes |

| | Example 26 | Example 27 | Example 28 | Example 29 | Example 30 |
|---|---|---|---|---|---|
| Deionized Water (g) | 63.6 | 63.6 | 61.4 | 68.05 | 65.05 |
| Ethanol (g) | 3.3 | 3.3 | 16.5 | 19.3 | 19.3 |
| 1,3-Butylene glycol (g) | | 20.0 | | | |
| Glycerol (g) | 20.0 | | 10.0 | | |
| Tetrasodium Glutamate Diacetate (g) | 0.1 | 0.1 | 0.1 | | |
| Potassium Dihydrogen Phosphate | | | | 0.5 | 0.5 |
| Sodium Hydroxide, 30% in Water | | | | 0.15 | 0.15 |
| Phospholipid Type PL1 (g) | 10.0 | 10.0 | 10.0 | 10.0 | 10.0 |
| Phospholipid Type PL1, wt% of phosphatidylcholine | 92 | 92 | 92 | 92 | 92 |
| Lupinus Albus Seed Extract (g) | 2.0 | 2.0 | 2.0 | 2.0 | 5.0 |
| Lupinus Albus Seed Extract Composition | (A) | (A) | (A) | (B) | (B) |
| Preservative* (g) | 1.0 | 1.0 | 0 | 0 | 0 |
| Particle size, nm | 309 | 288 | 195 | 188 | 261 |
| Physical Stability | 7 days @ 50°C | 7 days @ 50 °C | >16† days @ 50 °C | 84 days @ 40 °C | 84 days @ 40 °C |
| Was Example Composition Physically Stable? | No | No | Yes | Yes | Yes |

| | | | | | |
|---|---|---|---|---|---|
| (A) a Lupine Powder comprised of 38 wt% proteins, 10 wt% polysaccharides, 14 wt% lipids, 28 wt% fibers * Preservative comprised 79 wt% benzyl alcohol, 12 wt% benzoic acid, 8 wt% dehydroacetic acid † Test was stopped at the indicated number of days, with the sample still stable | | | | | |

### [Examples 28-30 outside of scope of present invention]

(A) a Lupine Powder comprised of 38 wt% proteins, 10 wt% polysaccharides, 14 wt% lipids, 28 wt% fibers
(B) a Lupine Powder comprised of 57 wt% proteins, 11 wt% polysaccharides, 13 wt% lipids, 5 wt% fibers
   * Preservative comprised 79 wt% benzyl alcohol, 12 wt% benzoic acid, 8 wt% dehydroacetic acid
   † Test was stopped at the indicated number of days, with the sample still stable

### Example 31. Skin Cream Containing Inventive "Liposome I" Lupine-Based Delivery System

| Ingredient | % weight |
|---|---|
| Composition of Example 2 | 3.00 |

| Phase A | |
|---|---|
| Deionized Water | Balance |
| Glycerol | 5.00 |
| Xanthan Gum | 0.65 |

| Phase B | |
|---|---|
| Dicaprylyl Ether | 5.00 |
| Octyldodecanol | 3.00 |
| Isomerized Safflower Acid (Clarinol® A-80) | 3.00 |
| Arachidyl Alcohol, Behenyl Alcohol, Arachidyl Glucoside (Montanov™ 202) | 3.00 |

| Phase C | |
|---|---|
| Glyceryl Caprylate | 1.00 |

The ingredients of phase A were added together, mixed and heated to 75 °C by using a Stephan UMC 5 mixer with emulsifying impeller at 300 to 400 rpm. The ingredients of phase B were added to a separate container, heated to 75 °C until melted, and mixed by using an overhead mechanical mixer. The phase B mixture was added to phase A while mixing, followed by homogenisation using the Stephan UMC 5 mixer with emulsifying impeller at 2700 rpm. The mixture was cooled to 60 °C and phase C was adding with gentle mixing. The mixture was further homogenised for a short time, cooled to 40 °C, and the Composition of Example 2 was added with gentle mixing until homogenous.

### Example 32. Skin Hydration Study of Cream Containing Inventive "Liposome I" Lupine-Based Delivery System

Measurements of skin hydration (i.e., the moisture content of stratum corneum) were made with a corneometer (Courage+Khazaka Model MPA 580 with probe type CM 825) according to the procedure of the operating manual. Five measurements were performed on each test area and the mean was used to define the skin hydration. The reported test below was conducted with 7 adult human panelists over the same test period.

Initial measurements of skin hydration were taken on a test area of the lower leg. The Skin Cream of Example 31 was applied in an amount of approximately 2 mg/cm² to the test area twice daily, morning and evening, for 28 days. Measurements of skin hydration were again made on the test area.

The mean average skin hydration for the 7 panelists increased 57%.

### Example 33. Placebo Cream Without Inventive Lupine-Based Delivery System

A Placebo Cream was prepared according to the composition and procedure of Example 31, except that the Lupine-Based Delivery System of Example 2 was not included.

### Example 34. Skin Hydration Study of Placebo Cream Without Inventive Lupine-Based Delivery System

The Placebo Cream of Example 33 was tested according to, over the same test period as, and with the same 7 adult human panelists as, Example 32.

The mean average skin hydration for the 7 panelists increased 45%.

### Example 35. Second Skin Cream Containing Inventive Lupine-Based Delivery System

| Ingredient | % weight |
|---|---|
| Phase A | |
| Deionized Water | balance |
| Glycerol | 3.0 |

| Phase B | |
|---|---|
| Sodium Behenoyl Lactylate | 2.0 |
| Cetearyl Alcohol | 3.0 |
| Glyceryl Stearate | 2.6 |
| Isopropyl Palmitate | 6.0 |
| Simmondsia Chinensis Seed Oil | 6.0 |

| Phase C | |
|---|---|
| Composition of Example 2 | 10.0 |
| Preservative of Phenoxyethanol (71-77 wt%), Benzoic Acid (11-13 wt%), Dehydroxyacetic Acid (6.5-7.5 wt%), Ethylhexylglycerin (1.4-11.4 wt%), Polyaminopropyl Biguanide (0.1-1.1 wt%) [Euxyl® K702 from Schülke & Mayr GmbH] | 0.5 |

The ingredients of phase A were added together, mixed and heated to 80 °C in accordance with Example 31. The ingredients of phase B were added to a separate container, mixed by using an overhead mechanical mixer and heated to 80 °C. The phase B mixture was added to phase A while mixing and homogenizing in accordance with Example 31. The mixture was cooled to 40 °C and ingredients of phase C were added with gentle mixing until the cream was homogeneous.

### Example 36. Third Skin Cream Containing Inventive Lupine-Based Delivery System

A Skin Cream is prepared according to the composition and procedure of Example 35, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 37. Fourth Skin Cream Containing Inventive Lupine-Based Delivery System

| Ingredient | % weight |
|---|---|
| Phase A | |
| Deionized Water | balance |
| Glycerol | 3.0 |
| Betaine | 1.5 |
| Xanthan Gum | 0.2 |

| Phase B | |
|---|---|
| Polyglyceryl-3 Methylglucose Distearate | 3.8 |
| Cetearyl Alcohol | 5.4 |
| Simmondsia Chinensis Seed Oil | 2.9 |
| Persea Gratissima Seed Oil | 4.2 |
| Mangifera Indica Seed Butter | 3.5 |

| Phase C | |
|---|---|
| Composition of Example 2 | 10.0 |
| Preservative of Phenoxyethanol (28.3-34.3 wt%), Benzyl Alcohol (28.3-34.3 wt%), Potassium Sorbate (17.0-20.6 wt%) [Euxyl® K700 from Schülke & Mayr GmbH] | 1.0 |

The Skin Cream was prepared according to the procedure of Example 35.

### Example 38. Fifth Skin Cream Containing Inventive Lupine-Based Delivery System

A Skin Cream is prepared according to the composition and procedure of Example 37, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 39. Sixth Skin Cream Containing Inventive Lupine-Based Delivery System

| Ingredient | % weight |
|---|---|
| Phase A | |
| Deionized Water | balance |
| Triticum Vulgare Kernel Flour | 6.0 |

| Phase B | |
|---|---|
| Glyceryl Stearate Citrate | 4.3 |
| Cetearyl Alcohol | 3.6 |
| Decyl Cocoate | 3.0 |
| Simmondsia Chinensis Seed Oil | 5.2 |
| Dicaprylyl Carbonate | 2.3 |
| Dicaprylyl Ether | 1.7 |

| Phase C | |
|---|---|
| Composition of Example 2 | 10.0 |
| Preservative of Benzyl Alcohol (79 wt%), Benzoic Acid (12 wt%), Dehydroacetic (8 wt% | 1.0 |

The Skin Cream was prepared according to the procedure of Example 35.

### Example 40. Seventh Skin Cream Containing Inventive Lupine-Based Delivery System

A Skin Cream is prepared according to the composition and procedure of Example 39, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 41. Eighth Skin Cream Containing Inventive Lupine-Based Delivery System

| Ingredient | % weight |
|---|---|
| Phase A | |
| Deionized Water | balance |
| Triticum Vulgare Kernel Flour | 6.0 |

| Phase B | |
|---|---|
| Brassica Campestris Seed Oil | 6.1 |
| Simmondsia Chinensis Seed Oil | 7.4 |
| Caprylic/Capric Triglyceride | 4.1 |
| Cetearyl Alcohol | 3.8 |
| Dicaprylyl Carbonate | 2.9 |
| Coco-Caprylate | 1.7 |

| Phase C | |
|---|---|
| Composition of Example 2 | 10.0 |
| Preservative of Benzyl Alcohol (79 wt%), Benzoic Acid (12 wt%), Dehydroacetic (8 wt%) | 1.0 |

The Skin Cream was prepared according to the procedure of Example 35.

### Example 42. Ninth Skin Cream Containing Inventive Lupine-Based Delivery System

A Skin Cream is prepared according to the composition and procedure of Example 41, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 43. First Serum Containing Inventive Lupine-Based Delivery System

| Ingredient | % weight |
|---|---|
| Phase A | |
| Deionized Water | Balance |
| Carbomer | 1.3 |

| Phase B | |
|---|---|
| Sodium Hydroxide, 10 wt% in Water | 3.6 |

| Phase C | |
|---|---|
| Sorbeth-30 | 5.0 |
| Preservative of Phenoxyethanol (71-77 wt%), Benzoic Acid (11-13 wt%), Dehydroxyacetic Acid (6.5-7.5 wt%), Ethylhexylglycerin (1.4-11.4 wt%), Polyaminopropyl Biguanide (0.1-1.1 wt% | 0.5 |
| Composition of Example 2 | 10.0 |

The ingredients of phase A were added together and mixed at room temperature by using an overhead mechanical mixer until the Carbomer was fully dissolved. The ingredient of phase B was added to the mixture for neutralization. Ingredients of phase C were then added in the order given with gentle mixing until the serum was homogeneous.

### Example 44. Second Serum Containing Inventive Lupine-Based Delivery System

A Serum is prepared according to the composition and procedure of Example 43, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 45. Tenth Skin Cream Containing Inventive Lupine-Based Delivery System

| Ingredient | % weight |
|---|---|
| Phase A | |
| Deionized Water | balance |
| Xanthan Gum | 0.9 |

| Phase B | |
|---|---|
| Isopropyl Myristate | 10.0 |
| Jojoba Oil | 1.0 |
| Cyclopentasiloxane | 5.0 |
| Cetearyl Alcohol (and) Dicetyl Phosphate (and) Ceteth-10 Phosphate | 6.0 |
| Bis-PEG/PPG-14/14 Dimethicone | 2.5 |
| Apricot Kernel Oil | 0.4 |
| Sweet Almond Oil | 0.6 |

| Phase C | |
|---|---|
| Composition of Example 2 | 3.0 |
| Propylene glycol, Diazolidinyl urea, Methylparaben, Propylparaben | 1.0 |
| Triethanolamine | as needed to achieve final pH 5.7 |

The ingredients of phase A were added together, mixed by using an overhead mechanical mixer, and heated to 85 °C. The ingredients of phase B were added to a separate container, mixed by using an overhead mechanical mixer, and heated to 85 °C. The phase B mixture was added to phase A and mixed for 3 minutes, cooled to 65 °C, and homogenized at 6000 rpm by using a high shear rotor-stator homogenizer for 3 minutes. The mixture was cooled with stirring to between 40 °C and 20 °C, and ingredients of phase C were then added in the order given with gentle mixing until the cream was homogeneous.

### Example 46. Eleventh Skin Cream Containing Inventive Lupine-Based Delivery System

A Skin Cream is prepared according to the composition and procedure of Example 45, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 47. Twelfth Skin Cream Containing Inventive Lupine-Based Delivery System

| Ingredient | % weight |
|---|---|
| Phase A | |
| Deionized Water | balance |
| Xanthan Gum | 0.9 |

| Phase B | |
|---|---|
| Mineral Oil | 12.5 |
| PEG-30 dipolyhydroxy stearate | 1.0 |
| Steareth-2 | 2.0 |
| Steareth 21 | 1.0 |
| Dimethicone | 4.5 |
| C12-15 Alkyl Benzoate | 4.5 |

| Phase C | |
|---|---|
| Composition of Example 2 | 3.0 |
| DMDM Hydantoin | 0.5 |

The Skin Cream was prepared according to the procedure of Example 45.

### Example 48. Thirteenth Skin Cream Containing Inventive Lupine-Based Delivery System

A Skin Cream is prepared according to the composition and procedure of Example 47, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 49. Fourteenth Skin Cream Containing Inventive Lupine-Based Delivery System

| Ingredient | % weight |
|---|---|
| Phase A | |
| Deionized Water | balance |
| Xanthan Gum | 0.2 |

| Phase B | |
|---|---|
| Octinoxate | 1.0 |
| Cetearyl Alcohol | 6.0 |
| Ceteareth-25 | 0.5 |

| Phase C | |
|---|---|
| Composition of Example 2 | 3.0 |
| Propylene Glycol, Diazolidinyl Urea, Methyparaben, Propylparaben | 1.0 |

The Skin Cream was prepared according to the procedure of Example 45.

### Example 50. Fifteenth Skin Cream Containing Inventive Lupine-Based Delivery System

A Skin Cream is prepared according to the composition and procedure of Example 49, except that the Composition of Example 2 is replaced by 5 wt% of any one of Example 1, Example 3, Example 4, Example 6, Example 7, Example 9, Example 10, Example 12, Example 14, Example 16, Example 21, Example 22, Example 23, Example 24, Example 25, Example 28, Example 29, or Example 30.

### Example 51. Sunscreen (SPF 15) Containing Inventive Lupine-Based Delivery System

| Ingredient | % weight |
|---|---|
| Phase A | |
| Deionized Water | balance |
| Acrylates/C10-30 Alkyl Acrylate Crosspolymer | 0.3 |
| Acrylate polymer | 1.0 |
| Triethanolamine | 1.0 |

| Phase B | |
|---|---|
| Octocrylene | 3.0 |
| Octisalate | 3.0 |
| Oxybenzone | 2.0 |
| Avobenzone | 1.0 |
| Stearic Acid | 2.0 |
| Glyceryl Monostearate SE | 3.0 |
| Dimethicone | 0.5 |
| C12-15 Alkyl Benzoate | 8.0 |

| Phase C | |
|---|---|
| Composition of any one of Example 1, Example 2, Example 3, Example 4, Example 6, Example 7, Example 9, Example 10, Example 12, Example 14, Example 16, Example 21, Example 22, Example 23, Example 24, Example 25, Example 28, Example 29, or Example 30 | 5.0 |
| Phenoxyethanol & Ethylhexylglycerin | 1.0 |

The Sunscreen is prepared according to the procedure of Example 45.

### Example 52. Hair Styling and Moisturizing Cream Containing Inventive Lupine-Based Delivery System

| Ingredient | % weight |
|---|---|
| Phase A | |
| Deionized Water | balance |
| Carbomer | 0.5 |
| Triethanolamine | 0.8 |
| Polyurethane-2 and Polymethyl Methacrylate | 5.0 |
| Polyquaternium-55 | 1.0 |

| Phase B | |
|---|---|
| Ceteareth-20 | 0.5 |
| Polysilicone-11 & Water & Laureth-12 & Phenoxyethanol & Ethylhexylglycerin | 4.0 |
| Dimethicone 100 Cst | 0.1 |
| PEG-12 Dimethicone | 5.0 |
| Jojoba oil | 2.0 |

| Phase C | |
|---|---|
| Composition of any one of Example 1, Example 2, Example 3, Example 4, Example 6, Example 7, Example 9, Example 10, Example 12, Example 14, Example 16, Example 21, Example 22, Example 23, Example 24, Example 25, Example 28, Example 29, or Example 30 | 5.0 |
| Phenoxyethanol & Ethylhexylglycerin | 1.0 |

The Hair Cream is prepared according to the procedure of Example 45.

### Example 53. Moisturizing BB Cream (Foundation) Containing Inventive Lupine-Based Delivery System

| Ingredient | % weight |
|---|---|
| Phase A | |
| Polysilicone-11 & Water & Laureth-12 & Phenoxyethanol & Ethylhexylglycerin | 5.0 |
| Dimethicone 50 Cst | 20.0 |
| PEG-10 Dimethicone | 2.0 |
| Dimethicone & PEG/PPG-18/18 Dimethicone | 2.0 |
| Disteardimonium Hectorite | 0.6 |
| Phase B | |
| Yellow Iron Oxide | 1.3 |
| Red Iron Oxide | 0.4 |
| Black Iron Oxide | 0.1 |
| Titanium Dioxide & Methicone | 6.6 |
| Mica | 6.6 |
| Dimethicone 50 Cst | 7.5 |
| PEG-10 Dimethicone | 2.0 |
| Phase C | |
| Deionized Water | balance |
| Glycerin | 5.0 |
| Butylene Glycol | 3.0 |
| Sodium Chloride | 1.0 |
| Phase D | |
| Composition of any one of Example 1, Example 2, Example 3, Example 4, Example 6, Example 7, Example 9, Example 10, Example 12, Example 14, Example 16, Example 21, | 3.0 |
| Example 22, Example 23, Example 24, Example 25, Example 28, Example 29, or Example 30 | |
| Phenoxyethanol & Ethylhexylglycerin | 1.0 |

The ingredients of phase A are added together, are mixed and heated to 85 °C, and the ingredients of phase B are added to a separate container, are mixed and heated to 85 °C in accordance with Example 45. The phase B mixture is added to phase A and is mixed for 3 minutes; the mixture is cooled to 65 °C, and is homogenized at 6000 rpm for 3 minutes in accordance with Example 45. Phase C ingredients are then added to the mixture and mixing is continued until homogeneous. The mixture is cooled with stirring to between 40 °C and 20 °C, and ingredients of phase D are then added with gentle mixing until the cream is homogeneous.

### Example 54. Hair Conditioner with Moisturization Containing Inventive Lupine-Based Delivery System

| Ingredient | % weight |
|---|---|
| Phase A | |
| Deionized Water | Balance |
| Polyquaternium-10 | 0.5 |
| Polyquaternium-4 | 0.7 |

| Phase B | |
|---|---|
| PEG 12 Dimethicone | 2.0 |
| Cyclopentasiloxane (and) Cetearyl Dimethicone/Vinyl Dimethicone Crosspolymer | 4.5 |
| Composition of any one of Example 1, Example 2, Example 3, Example 4, Example 6, Example 7, Example 9, Example 10, Example 12, Example 14, Example 16, Example 21, Example 22, Example 23, Example 24, Example 25, Example 28, Example 29, or Example 30 | 3.0 |
| Phenoxyethanol & Ethylhexylglycerin | 1.0 |

The ingredients of phase A are added together at room temperature and mixed by using an overhead mechanical mixer until homogeneous. The ingredient of phase B are added together in a separate container at room temperature and mixed by using an overhead mechanical mixer until homogeneous. Phase B is added to phase A and mixed until the conditioner is homogeneous.

The invention also relates to the following numbered items:
1. A composition comprising at least one Lupine Composition and at least one Delivery System.
2. The composition of Item 1 wherein the Lupine Composition comprises a Lupine Extract.
3. The composition of Item 2 wherein the Lupine Extract comprises a seed extract of Lupinus albus.
4. The composition of Item 1 wherein the Lupine Composition comprises Lupine Powder.
5. The composition of Item 4 wherein the Lupine Powder comprises a seed of genus Lupinus.
6. The composition of Item 5 wherein the seed comprises Lupinus albus.
7. The composition of Item 1 wherein the Lupine Composition comprises materials derived from any one or more parts of, or whole plant of, any plant within genus Lupinus.
8. The composition of Item 7 wherein the material comprises at least one of hydrolyzed Lupine extracts, partially hydrolyzed Lupine extracts, a substance derived from lupine and combinations thereof.
9. The composition of Item 1 wherein the Delivery System comprises at least one member selected from the group consisting of Cerasome, Liposome I and Liposome II.
10. The composition of Item 9 wherein the Delivery System comprises Liposome I.
11. The composition of Item 10 wherein the Liposome I comprises at least one of Phospholipids of type PL2.
12. The composition of Item 9 further comprising at least one solvent selected from the group consisting of ethanol, 1,2-propanediol, 1,3-propanediol, 1,3-butylene glycol, and glycerol.
13. A Personal Care product comprising the composition of Item 1.
14. A Cosmetic product comprising the composition of Item 1.
15. The composition of Item 1 wherein the composition is physically stable.
16. The composition of Item 9 wherein the composition is physically stable.
17. The composition of Item 1 further comprising at least one chelating agent.
18. The composition of Item 1 further comprising at least one preservative.
19. Use of at least one Delivery System to yield a Lupine-Based Delivery System composition that is physically stable.

## Claims

1. A composition comprising at least one Lupine Composition and at least one Delivery System
wherein at least a portion of the Lupine Composition is encapsulated in the Delivery System and the Delivery System comprises at least one member selected from the group consisting of Cerasome, Liposome I and Liposome II, and wherein
Cerasome denotes an artificially prepared vesicle made of at least one lipid bilayer and the Cerasome composition comprises
i) one or more naturally-derived or synthetic ceramides and/or naturally-derived or synthetic glycosphingolipids in an amount from 0.1 wt% to 15 wt%,
ii) the at least one Lupine Composition in an amount from 0.1 wt% to 15 wt%,
iii) at least one solvent, comprising at least one alcohol, glycol, or triol, in an amount from 5 wt% to 30 wt% and
iv) water in an amount of at least 30 wt%;
Liposome I denotes an artificially prepared vesicle made of at least one lipid bilayer, wherein the Delivery System comprises naturally-derived or synthetic phospholipids of type PL2, which comprise phosphatidylcholine, phosphatidylethanolamine and phosphatidic acid, wherein the content of phosphatidylcholine is from 10 wt% to 60 wt%, based on the total weight of the phospholipids and a Liposome I composition comprises
i) one or more of the naturally-derived or synthetic phospholipids of type PL2, in an amount from 0.1 wt% to 25 wt%,
ii) the at least one Lupine Composition in an amount from 0.1 wt% to 15 wt%,
iii) at least one solvent, comprising at least one alcohol, glycol or triol, in an amount from 5 wt% to 50 wt%,
iv) water in an amount of at least 30 wt%; and wherein
- if the composition comprises ethanol, the amount is from 5 wt% to 15 wt%,
- if the composition comprises 1,2-propanediol or 1,3-propanediol, the amount is from 15 wt% to 25 wt%,
- if the composition comprises 1,3-butylene glycol, the amount is from 5 wt% to 25 wt%,
- if the composition comprises glycerol, the amount is from 15 wt% to 35 wt%; and
Liposome II denotes an artificially prepared vesicle made of at least one lipid bilayer, wherein the Delivery System comprises naturally-derived or synthetic phospholipids of type PL1, which comprise phosphatidylcholine, phosphatidylethanolamine and phosphatidic acid, wherein the amount of phosphatidylcholine is from 70 wt% to 97 wt%, based on the total weight of the phospholipids and a Liposome II composition comprises
i) one or more of the naturally-derived or synthetic phospholipids of type PL1, in an amount from about 1 wt% to about 25 wt%,
ii) the at least one Lupine Composition in an amount from about 0.1 wt% to about 15 wt%,
iii) at least one solvent, comprising at least one alcohol, glycol or triol, in an amount from about 5 wt% to about 50 wt%,
iv) water in an amount of at least 30 wt%, and wherein
- if the solvent comprises at least one solvent selected from the group consisting of ethanol, 1,2-propanediol, 1,3-propanediol, 1,3-butylene glycol, and glycerol, the amount of the selected solvent is from 5 wt% to 25 wt%, based on the total weight of the composition;
and wherein if not otherwise mentioned amounts in wt% are based on the total weight of the composition.

2. The composition of Claim 1 wherein the Lupine Composition comprises a Lupine Extract.

3. The composition of Claim 2 wherein the Lupine Extract comprises a seed extract of Lupinus albus.

4. The composition of Claim 1 wherein the Lupine Composition comprises Lupine Powder.

5. The composition of Claim 4 wherein the Lupine Powder comprises a seed of genus Lupinus.

6. The composition of Claim 5 wherein the seed comprises Lupinus albus.

7. The composition of Claim 1 wherein the Lupine Composition comprises materials derived from any one or more parts of, or whole plant of, any plant within genus Lupinus.

8. The composition of Claim 7 wherein the material comprises at least one of hydrolyzed Lupine extracts, partially hydrolyzed Lupine extracts, a substance derived from lupine and combinations thereof.

9. The composition of Claim 1 wherein the Delivery System comprises Liposome I.

10. The composition of Claim 1 comprising at least one solvent selected from the group consisting of ethanol, 1,2-propanediol, 1,3-propanediol, 1,3-butylene glycol, and glycerol.

11. A Personal Care product comprising the composition of Claim 1.

12. A Cosmetic product comprising the composition of Claim 1.

13. The composition of Claim 1 wherein the composition is physically stable.

14. The composition of Claim 1 further comprising at least one member selected from the group consisting of chelating agent and preservative.

15. Use of at least one Delivery System according to claim 1 to yield a Lupine-Based Delivery System composition that is physically stable.

## Patentansprüche

1. Zusammensetzung, umfassend mindestens eine Lupinenzusammensetzung und mindestens ein Zuführungs system,
wobei mindestens ein Teil der Lupinenzusammensetzung in dem Zuführungssystem verkapselt ist und das Zuführungssystem mindestens ein Mitglied aus der Gruppe bestehend aus Cerasom, Liposom I und Liposom II umfasst und wobei
Cerasom ein künstlich hergestelltes Vesikel aus mindestens einer Lipid-Doppelschicht bezeichnet und die Cerasomzusammensetzung
i) ein oder mehrere natürliche oder synthetische Ceramide und/oder natürliche oder synthetische Glykosphingolipide in einer Menge von 0,1 Gew.-% bis 15 Gew.-%,
ii) die mindestens eine Lupinenzusammensetzung in einer Menge von 0,1 Gew.-% bis 15 Gew.-%,
iii) mindestens ein Lösungsmittel, das mindestens einen Alkohol, mindestens ein Glykol oder mindestens ein Triol umfasst, in einer Menge von 5 Gew.-% bis 30 Gew.-% und
iv) Wasser in einer Menge von mindestens 30 Gew.-%
umfasst;
Liposom I ein künstlich hergestelltes Vesikel aus mindestens einer Lipid-Doppelschicht bezeichnet, wobei das Zuführungssystem natürliche oder synthetische Phospholipide vom Typ PL2 umfasst, die Phosphatidylcholin, Phosphatidylethanolamin und Phosphatidinsäure umfassen, wobei der Gehalt an Phosphatidylcholin 10 Gew.-% bis 60 Gew.-%, bezogen auf das Gesamtgewicht der Phospholipide, beträgt und eine Liposom-I-Zusammensetzung
i) ein oder mehrere natürliche oder synthetische Phospholipide des Typs PL2 in einer Menge von 0,1 Gew.-% bis 25 Gew.-%,
ii) die mindestens eine Lupinenzusammensetzung in einer Menge von 0,1 Gew.-% bis 15 Gew.-%,
iii) mindestens ein Lösungsmittel, das mindestens einen Alkohol, mindestens ein Glykol oder mindestens ein Triol umfasst, in einer Menge von 5 Gew.-% bis 50 Gew.-% und
iv) Wasser in einer Menge von mindestens 30 Gew.-%
umfasst und wobei
- dann, wenn die Zusammensetzung Ethanol umfasst, die Menge 5 Gew.-% bis 15 Gew.-% beträgt,
- dann, wenn die Zusammensetzung 1,2-Propandiol oder 1,3-Propandiol umfasst, die Menge 15 Gew.-% bis 25 Gew.-% beträgt,
- dann, wenn die Zusammensetzung 1,3-Butylenglykol umfasst, die Menge 5 Gew.-% bis 25 Gew.-% beträgt,
- dann, wenn die Zusammensetzung Glycerin umfasst, die Menge 15 Gew.-% bis 35 Gew.-% beträgt; und
Liposom II ein künstlich hergestelltes Vesikel aus mindestens einer Lipid-Doppelschicht bezeichnet, wobei das Zuführungssystem natürliche oder synthetische Phospholipide vom Typ PL1 umfasst, die Phosphatidylcholin, Phosphatidylethanolamin und Phosphatidinsäure umfassen, wobei der Gehalt an Phosphatidylcholin 70 Gew.-% bis 97 Gew.-%, bezogen auf das Gesamtgewicht der Phospholipide, beträgt und eine Liposom-II-Zusammensetzung
i) ein oder mehrere natürliche oder synthetische Phospholipide des Typs PL1 in einer Menge von etwa 1 Gew.-% bis etwa 25 Gew.-%,
ii) die mindestens eine Lupinenzusammensetzung in einer Menge von etwa 0,1 Gew.-% bis etwa 15 Gew.-%,
iii) mindestens ein Lösungsmittel, das mindestens einen Alkohol, mindestens ein Glykol oder mindestens ein Triol umfasst, in einer Menge von etwa 5 Gew.-% bis etwa 50 Gew.-% und
iv) Wasser in einer Menge von mindestens 30 Gew.-%
umfasst und wobei
- dann, wenn das Lösungsmittel mindestens ein Lösungsmittel aus der Gruppe bestehend aus Ethanol, 1,2-Propandiol, 1,3-Propandiol, 1,3-Butylenglykol und Glycerin umfasst, die Menge des gewählten Lösungsmittels 5 Gew.-% bis 25 Gew.-%, bezogen auf das Gesamtgewicht der Zusammensetzung, beträgt;
und wobei Mengen in Gew.-% auf dem Gesamtgewicht der Zusammensetzung basieren, sofern nicht anders angegeben.

2. Zusammensetzung nach Anspruch 1, wobei die Lupinenzusammensetzung ein Lupinenextrakt umfasst.

3. Zusammensetzung nach Anspruch 2, wobei das Lupinenextrakt ein Samenextrakt von Lupinus albus umfasst.

4. Zusammensetzung nach Anspruch 1, wobei die Lupinenzusammensetzung ein Lupinenpulver umfasst.

5. Zusammensetzung nach Anspruch 4, wobei das Lupinenpulver einen Samen der Gattung Lupinus umfasst.

6. Zusammensetzung nach Anspruch 5, wobei der Samen Lupinus albus umfasst.

7. Zusammensetzung nach Anspruch 1, wobei die Lupinenzusammensetzung Materialien umfasst, die sich von einem oder mehreren Teilen oder der ganzen Pflanze einer Pflanze der Gattung Lupinus ableiten.

8. Zusammensetzung nach Anspruch 7, wobei das Material mindestens eines von hydrolysierten Lupinenextrakten, teilhydrolysierten Lupinenextrakten, einer von Lupinen abgeleiteten Substanz und Kombinationen davon umfasst.

9. Zusammensetzung nach Anspruch 1, wobei das Zuführungssystem Liposom I umfasst.

10. Zusammensetzung nach Anspruch 1, umfassend mindestens ein Lösungsmittel aus der Gruppe bestehend aus Ethanol, 1,2-Propandiol, 1,3-Propandiol, 1,3-Butylenglykol und Glycerin.

11. Körperpflegeprodukt, umfassend die Zusammensetzung nach Anspruch 1.

12. Kosmetikprodukt, umfassend die Zusammensetzung nach Anspruch 1.

13. Zusammensetzung nach Anspruch 1, wobei die Zusammensetzung physikalisch stabil ist.

14. Zusammensetzung nach Anspruch 1, ferner umfassend mindestens ein Mitglied aus der Gruppe bestehend aus Chelatbildner und Konservierungsmittel.

15. Verwendung mindestens eines Zuführungssystem nach Anspruch 1 zum Erhalt einer Zuführungssystemzusammensetzung auf Lupinenbasis, die physikalisch stabil ist.

## Revendications

1. Composition comprenant au moins une composition de lupin et au moins un système d'administration dans laquelle au moins une partie de la composition de lupin est encapsulée dans le système d'administration et le système d'administration comprend au moins un membre choisi dans le groupe constitué d'un cérasome, un liposome I et un liposome II, et dans laquelle un cérasome désigne une vésicule préparée artificiellement constituée d'au moins une bicouche lipidique et la composition de cérasome comprend
i) un ou plusieurs céramides d'origine naturelle ou synthétiques et/ou des glycosphingolipides d'origine naturelle ou synthétiques en une quantité de 0,1 % en poids à 15 % en poids,
ii) l'au moins une composition de lupin en une quantité de 0,1 % en poids à 15 % en poids,
iii) au moins un solvant, comprenant au moins un alcool, un glycol ou un triol, en une quantité de 5 % en poids à 30 % en poids et
iv) de l'eau en une quantité d'au moins 30 % en poids ;
le liposome I désigne une vésicule préparée artificiellement constituée d'au moins une bicouche lipidique, le système de libération comprenant des phospholipides d'origine naturelle ou synthétiques de type PL2, qui comprennent la phosphatidylcholine, la phosphatidyléthanolamine et l'acide phosphatidique, dans laquelle la teneur en phosphatidylcholine est de 10 % en poids à 60 % en poids, sur la base du poids total des phospholipides et une composition de liposome I comprend
i) un ou plusieurs des phospholipides d'origine naturelle ou synthétiques de type PL2, en une quantité de 0,1 % en poids à 25 % en poids,
ii) l'au moins une composition de lupin en une quantité de 0,1 % en poids à 15 % en poids,
iii) au moins un solvant, comprenant au moins un alcool, un glycol ou un triol, en une quantité de 5 % en poids à 50 % en poids,
iv) de l'eau en une quantité d'au moins 30 % en poids ; et dans laquelle
- si la composition comprend de l'éthanol, la quantité est de 5 % en poids à 15 % en poids,
- si la composition comprend du 1,2-propanediol ou du 1,3-propanediol, la quantité est de 15 % en poids à 25 % en poids,
- si la composition comprend du 1,3-butylène glycol, la quantité est de 5 % en poids à 25 % en poids,
- si la composition comprend du glycérol, la quantité est de 15 % en poids à 35 % en poids ; et
le liposome II désigne une vésicule artificielle constituée d'au moins une bicouche lipidique, le système de libération comprenant des phospholipides d'origine naturelle ou synthétiques de type PL1, comprenant de la phosphatidylcholine, de la phosphatidyléthanolamine et de l'acide phosphatidique, la quantité de phosphatidylcholine allant de 70 % en poids à 97 % en poids, basé sur le poids total des phospholipides et une composition de Liposome II comprend
i) un ou plusieurs des phospholipides d'origine naturelle ou synthétiques de type PL1, en une quantité d'environ 1 % en poids à environ 25 % en poids,
ii) l'au moins une composition de lupin en une quantité d'environ 0,1 % en poids à environ 15 % en poids,
iii) au moins un solvant, comprenant au moins un alcool, un glycol ou un triol, en une quantité d'environ 5 % en poids à environ 50 % en poids,
iv) de l'eau en une quantité d'au moins 30 % en poids, et dans laquelle
- si le solvant comprend au moins un solvant choisi dans le groupe constitué de l'éthanol, le 1,2-propanediol, le 1,3-propanediol, le 1,3-butylène glycol et le glycérol, la quantité du solvant choisi est de 5 % en poids à 25 % en poids, sur la base du poids total de la composition ;
et dans laquelle, sauf indication contraire, les quantités mentionnées en % en poids sont basées sur le poids total de la composition.

2. Composition selon la revendication 1, dans laquelle la composition de lupin comprend un extrait de lupin.

3. Composition selon la revendication 2, dans laquelle l'extrait de lupin comprend un extrait de graines de Lupinus albus.

4. Composition selon la revendication 1, dans laquelle la composition de lupin comprend de la poudre de lupin.

5. Composition selon la revendication 4, dans laquelle la poudre de lupin comprend une graine du genre Lupinus.

6. Composition selon la revendication 5, dans laquelle la graine comprend Lupinus albus.

7. Composition selon la revendication 1, dans laquelle la composition de lupin comprend des matériaux dérivés d'une ou plusieurs parties quelconques de, ou une plante entière de, une plante quelconque dans le genre Lupinus.

8. Composition selon la revendication 7, dans laquelle le matériau comprend au moins l'un des extraits de lupin hydrolysés, des extraits de lupin partiellement hydrolysés, une substance dérivée du lupin et des combinaisons de ceux-ci.

9. Composition selon la revendication 1, dans laquelle le système d'administration comprend un liposome I.

10. Composition selon la revendication 1 comprenant au moins un solvant choisi dans le groupe constitué par l'éthanol, le 1,2-propanediol, le 1,3-propanediol, le 1,3-butylène glycol et le glycérol.

11. Produit de soin personnel comprenant la composition de la revendication 1.

12. Produit cosmétique comprenant la composition de la revendication 1.

13. Composition selon la revendication 1, dans laquelle la composition est physiquement stable.

14. Composition selon la revendication 1, comprenant en outre au moins un membre choisi dans le groupe constitué d'agent chélateur et de conservateur.

15. Utilisation d'au moins un système d'administration selon la revendication 1 pour obtenir une composition de système d'administration à base de lupin qui est physiquement stable.
